(19) Europäisches Patentamt
European Patent Office
Office européen des brevets

(11) Publication number: **0 497 464 A1**

(12) **EUROPEAN PATENT APPLICATION**

(21) Application number: **92300344.6**

(22) Date of filing: **15.01.92**

(51) Int. Cl.[5]: **C12Q 1/68**, C12Q 1/70, C12Q 1/06

(30) Priority: **31.01.91 US 649569**

(43) Date of publication of application: **05.08.92 Bulletin 92/32**

(84) Designated Contracting States: **DE FR GB IT NL**

(71) Applicant: **AMOCO CORPORATION**
**200 East Randolph Drive**
**Chicago Illinois 60601(US)**

(72) Inventor: **Müller, Uwe Richard**
**11714 River Road**
**Plano, Illinois 60545(US)**
Inventor: **Cruickshank, Kenneth Alexander**
**128 Robin Hill Drive**
**Naperville, IL 60540(US)**

(74) Representative: **Laredo, Jack Joseph et al**
**Elkington & Fife Prospect House 8 Pembroke Road**
**Sevenoaks, Kent TN13 1XR(GB)**

(54) **Rapid microbial diagnostics by in situ hybridization in aqueous suspension.**

(57) The invention provides aqueous phase in situ hybridization processes using labeled nucleic acid probes by which single cell microorganisms can be identified and quantified. In a first process step, microorganisms are fixed and their cell walls permeabilized for passage therethrough of oligonucleotides yet cell morphology is not changed. Thereafter, the cells are subjected to hybridization conditions followed by detection of the probe labels. Bacteria and yeasts are presently preferred starting cells. The product hybridized cells are new and are useful as diagnostic agents.

EP 0 497 464 A1

## Field of the Invention

This invention relates to the *in situ* hybridization of nucleic acid probes with nucleic acids within cytological structures.

## Background of the Invention

Nucleic acid hybridization involves the formation of the double helix from two single stranded complementary polymeric nucleotides, each comprised of either an oligomer or a polymer. Such two nucleotides can be either two single stranded DNA molecules, or one single stranded DNA molecule and one single stranded RNA molecule. Hybridization can be used to analyze relationships between nucleic acid sequences and is applied to a wide range of biological problems.

In hybridization, it is common to employ a probe that is comprised of a nucleic acid portion and a chemically bound label portion. Such a probe is used to detect, by complementary base pairing, another or target nucleic acid molecule that has a complementary or homologous nucleotide sequence. The label portion of a probe permits detection of the hybridization reaction or reaction product. The label portion of a probe molecule can be considered to be at least one moiety that incorporates, or contains, for example, a fluorophore, a chromogen, a radioisotope, an enzyme, biotin, a hapten, an avidin, an antibody, or the like.

Hybridization has usually been carried out with the target RNA or DNA molecules or fragments thereof bound to a substrate, such as a nitrocellulose or nylon membrane, or a vitreous surface, such as glass or porcelain. The membrane or substrate with bound molecules is then washed with a solution of an appropriate DNA or RNA probe which binds to the DNA or RNA nucleic acid target molecule or fragment of interest by complementary base pairing. The resulting hybrid is detected or quantified using the label portion of the probe.

Although *in situ* hybridization of a nucleic acid probe to nucleic acids within cytological preparations is known to the prior art (see, for example, M.L. Pardue, "In situ Hybridization" in "Nucleic Acid Hybridisation A Practical Approach" at pp. 179-202 (1985) published by IRL Press), so far as now known, only the publications by Pace et al. relate to *in situ* hybridization within bacteria and yeasts (Giovannoni, S.J., DeLong, E.F., Olsen, G.J. and Pace, N.R. (1988) "Phylogenetic Group-Specific Oligodeoxynucleotide Probes for Identification of Single Microbial Cells," J. Bacteriol. 170: 720-726 and DeLong, E.F., Wickham, G.S. and Pace, N.R. (1989) "Phylogenetic Stains: Ribosomal RNA-based Probes for the Identification of Single Cells," Science 243: 1360-1363).

Pace et al. phylogenetically characterized specific prokaryotic cells in admixture with other prokaryotic and/or eukaryotic cells with universal and species-specific probes directed towards 16S rRNA using isotopically labeled probes and also fluorescein- or rhodamine-labeled probes. The Pace et al. method involved first fixing cells in aqueous media and then mounting the cells on slides where a multi-step hybridization and washing procedure was then performed. The total time required was lengthy (evidently at least about 6 hours) and an initial cell concentration of about $10^7$ cells per ml was needed.

The Pace et al. hybridization procedure suffers from various disadvantages. For one thing, the procedural protocol is slow, requiring between 5 and 16 hours just for the hybridization. A relatively high cell concentration is needed for detection. The laborious processing requires many steps. The Pace et al. format, in fact, is unsuitable for practical application in a medical diagnostics laboratory.

For another thing, the Pace et al. hybridization procedure requires fixing and then mounting the fixed cells on slides so that such procedure is limited to an observation of the fixed cells on slides for purposes of measuring the hybridization event. Microscopy or possibly an imaging system must be used. Individual cells must be examined and evaluated. At best, microscopic examination and evaluation can be a laborious procedure.

For another thing, the Pace et al. hybridization procedure is only effective as an identification tool when cellular ribosomal RNA (rRNA) targets are involved. Thus, in order to see a cell under the fluorescence microscope, one needs about 10,000 fluorophores per cell. Pace et al. use short, oligomeric DNA probes that are labeled with one fluorophore per probe molecule. The only stable nucleic acid target present in cells at such a high number is ribosomal RNA. Messenger RNA is generally present in lower copy number (about 1000 molecules, or less). Plasmid DNA molecules could be present with up to about 1000 copies while the bacterial chromosome is present in only one copy. Also, there are no sequences on the chromosomal or plasmid DNA that are repeated to anywhere near the number needed to bind 10,000 probes of a unique sequence.

However, for various purposes, hybridization of rRNA nucleic acids is not sufficient for microorganism diagnostic (that is, microorganism detection, identification, characterization, quantification and/or the like)

purposes. For example, at the present time, there is no known rRNA nucleotide sequence which is unique in Shigellae compared to E. coli. Thus, so far as now known, neither species of such bacteria can be distinguished from the other by an rRNA nucleotide sequence. However, DNA nucleotide sequences do distinguish Shigellae from E. coli. Such a distinction has practical consequences in medicine since Shigellae are pathogens which can cause a life-threatening infection which is differently treated from an infection caused by E. coli.

The Pace et al. hybridization procedure involving fixing and slide mounting cannot be converted to a more rapid diagnostic technique such as described herein where aqueous cell dispersions are used without any slide mounting. For one thing, although centrifuging is not taught by Pace et al., the cell processing procedure described by Pace et al. results in cells which are largely lost during centrifugation. For another thing, the microscopic or the autoradiographic detection procedures taught by Pace et al. for slide mounted cells are not easily adapted for rapid, accurate usage compared with aqueous dispersions of hybridized cells.

Thus, a new and improved in situ hybridization procedure is needed which will overcome the shortcomings of the prior art and provide a rapid, practical procedure for diagnosis of microorganisms, such as bacteria, yeasts, and the like, without substrate mounting.

## Summary of the Invention

The present invention relates to new and improved methods for determining the presence of microorganisms by in situ hybridization in aqueous suspension. Cellular nucleic acids are hybridized within their producing single cell microorganisms with complementary nucleic acid probes.

The invention also relates to fixed and permeabilized microorganisms containing hybridized nucleic acid components which are produced by such methods and which are useful for diagnostic purposes.

So far as now known, no one has heretofore succeeded in accomplishing the in situ hybridization under aqueous liquid phase suspension conditions of cellular nucleic acids in fixed and permeabilized but unmounted microorganisms with labeled nucleic acid probes. As described herein, a simple and reliable procedure is provided which can be rapidly performed so that single cell microorganisms can be internally hybridized and then diagnosed through probe label detection.

More particularly, the present invention provides a method for determining the presence of a microorganism while the microorganism is dispersed in an aqueous medium. This method involves the steps of:

(a) first contacting the microorganism while in an aqueous dispersion with a water miscible liquid treating composition which fixes such microorganism and also permeabilizes the cell wall thereof to an extent sufficient to permit penetration therethrough of an oligonucleotide without appreciably altering the cellular morphology of such microorganism;

(b) preferably separating the resulting so contacted microorganism from the treating composition;

(c) preferably resuspending the so separated microorganisms in an aqueous medium; and

(d) secondly contacting such suspended microorganisms with nucleic acid probes at least one of which is labeled.

A suitable probe incorporates a nucleotide sequence that is complementary to a particular target nucleotide sequence that exists in the cellular interior of such microorganism, thereby to cause hybridization of such cellular nucleotide sequence with such probe as a result of such second contacting. Thereafter, the presence in such microorganism of such internally hybridized nucleic acid component(s) is/are detected using probe labels.

In one aspect, the present invention provides an aqueous liquid phase process for identifying the presence of a particular microorganism, such as a microorganism class, phylogenetic group, species, or the like. In the process, the microorganism, either alone or in combination with other different microorganisms, is dispersed in an aqueous medium and preferably fixed and permeabilized as taught herein before being contacted with at least one type of nucleic acid probe (preferably an oligonucleotide probe). The probe(s) is/are targeted for at least one particular nucleotide grouping that is known to be characteristically present in at least one type of cellular nucleic acid existing in the microorganism to be identified. After a time period has elapsed within which the hybridization between the probe oligonucleotide(s) and the cellular nucleotide grouping(s) would occur within the microorganism to be identified under the particular temperature and liquid phase conditions selected, usually about 10 minutes to about 2 hours, the resulting microorganisms are examined to detect whether or not hybridization has occurred therewithin. Hybridization produces a detectable level of probe labels within the microorganism cell so that label detection identifies the presence of the particular target nucleotide sequence(s) to which the probe(s) was/were targeted.

In another and related aspect, the present invention provides an aqueous liquid phase process wherein

the quantity of such a particular microorganism is determined. The microorganism can either be alone or in admixture with other different microorganisms, tissue fragments, or the like. The procedural steps are similar to those employed in the foregoing process except that during the terminal detection or probe-contacted cell examination step, the probe label intensity caused by hybrids being present in the cell is measured. From that measurement, an estimate of the quantity of the particular hybrid-containing microorganism is derived.

The processes of this invention are well adapted to the formation and detection of cellular hybrids of nucleic acids which are present in relatively low copy numbers per cell. In another aspect, the present invention provides techniques for identifying and characterizing DNA and RNA nucleic acid structures which are present in a microorganism in low copy numbers including even only single copy structures (such as chromosomal DNA and the like).

The present invention further provides a new and very useful class of novel hybridized microorganisms. Members of this class are each a water-dispersable, fixed microorganism whose cell wall structure has been permeabilized as described herein, but whose morphology is unchanged. Also such cell structure is sufficiently stable to undergo centrifuging (a) without morphological change and (b) without significant cell loss. In addition, the cell walls of such members have been permeated (that is, penetrated) by oligonucleotide probes which, as a result of the complementary nucleotide sequence(s) chosen for inclusion in the oligonucleotide portion of such probes, have been hybridized with at least one particular nucleotide sequence which is present in that microorganism. These novel hybridized microorganisms are never bound to a substrate during processing in accord with this invention which allows their use in a variety of different detection methods in addition to microscopy. As a consequence, cellular nucleic acids, such as those which are present in low copy numbers, can be targeted for hybridization with a probe or, probes, such as, for example, messenger RNA (mRNA), transfer RNA (tRNA), extra-chromosomal DNA (exc-DNA), including plasmid DNA (pla-DNA) and bacteriophage DNA, (pha-DNA), and chromosomal DNA (chr-DNA).

One preferred group of this novel class of hybridized microorganisms comprise microorganisms which have been fixed and permeabilized in accord with this invention using an aqueous solution comprising about 72 volume percent methanol, about 3.6 volume percent formaldehyde, and about 24.4 volume percent water on a 100 volume percent total liquid basis.

Presently most preferred members of the class are detectable microorganisms which have been so fixed, permeabilized and hybridized, and wherein the cellular hybridized nucleic acid component is selected from the group consisting of RNA and DNA such as the above exemplified types which are present in low copy numbers per cell.

Nucleic acid probes useful in the practice of this invention can be directly or indirectly labeled probes known to the prior art that are prepared by procedures known to the prior art. Presently preferred probes are oligonucleotides. Label moieties include fluorophores, chromogens, enzymatically active proteins, radioisotopes, and the like. Presently preferred probes are fluorophore labeled. It is considered a feature and an advantage of this invention that prior art probes and synthetic methods can be used in the practice of this invention, thereby avoiding the need to develop new and perhaps specialized probe technology suitable for use in the practice of this invention.

Hybrid detection procedures and equipment employed in the practice of this invention can be those known to the prior art. It is considered a feature and an advantage of the present invention that such prior art detection procedures and equipment can be used in the practice of the invention, thereby avoiding the need to develop new and perhaps specialized detection technology suitable for use in the practice of this invention.

The present invention provides in one aspect a technique for the simultaneous detection of multiple single cell microbial pathogens within a single specimen. In such a technique, a plurality of different oligonucleotide probes, each preferably differently fluorescently colored with its label moiety or moieties, are used.

Because of the characteristic lower copy frequency of occurrence of nucleic acids, such as mRNA, exc-DNA and chr-DNA, relative to, for example, the occurrence of rRNA in microorganisms, it is presently preferred to have incorporated into probes used in the practice of this invention (particularly when hybridization of such low copy frequency nucleic acids materials is contemplated) more than one label moiety per probe molecule for purposes of magnifying the capacity for hybrid detection and measurement (quantification). In, for example, the presently preferred fluorophore-containing oligonucleotide probes, it is presently preferred to use probes containing at least two fluorophore moieties per probe molecule.

In another aspect, the present invention makes possible various new and very useful diagnostic procedures for single cell microorganism identification and/or characterization.

By preliminarily appropriately pretreating microbial cells before fixation and permeabilization, the

amount of a cellular target nucleic acid material can be increased, thereby increasing the number of hybrids which can be formed within such cells from a given probe and thereby enhancing detectability.

In a further aspect, the present invention provides practical diagnostic kits which are usable in medical laboratories or the like for the diagnosis of microorganisms, particularly pathogens. Such kits utilize the inventive processes and products. One such kit, for example, provides a rapid means for qualitatively identifying the presence or absence of a particular microbial species, group, or the like in a sample fluid. Another such kit, for example, provides a rapid means for quantitatively identifying the extent of the presence of a particular microbial species, group, or the like, in a sample fluid.

The kits of the present invention feature simplicity, reliability, accuracy and rapidity of usage.

Advantageously, when, for example, this invention is practiced with fluorophore labeled probes, the inventive methods are rapidly learnable by laboratory personnel who are already familiar with histological/cytological staining materials for microbial cells and the like.

It is a feature of this invention that the present process and product technology provided is well suited for automated usage.

It is another feature of the present invention that an initial suspended cell concentration of only about 10 internally hybridized cells per µl (equivalent to about $10^4$ such cells per ml) is needed for cell diagnosis detection, identification, characterization, quantification and/or the like by microscopy using for example, target cellular rRNA or like cellular nucleic acid material and fluorophore labeled oligonucleotide probes with complementary nucleotide sequences within a total time of not more than about 2 hours and preferably not more than about 1.5 hour. Even lower such cell concentrations can be used with other detection methods.

Advantageously, the present invention is practiced without culture and without "in vitro" amplification (that is, without increase in copy number of characterizable or identifiable cellular nucleotide sequences).

Other and further objects, aims, purposes, features, advantages, embodiments, applications, and the like will be apparent to those skilled in the art from the present specification taken with the accompanying drawings and the appended claims.

## Brief Description of the Drawings

In the drawings:

Fig. 1 is a flow sheet illustrating an embodiment of a process for practicing the present invention;

Fig. 2 is another flow sheet illustrating a presently preferred embodiment of a process for practicing the present invention;

Fig. 3 is another flow sheet illustrating an embodiment of a process for practicing the present invention using fluorophore labeled probes;

Fig. 4 is another flow sheet illustrating an embodiment of a process for practicing the present invention using radioisotope-labeled probes;

Fig. 5 is another flow sheet illustrating embodiments of processes for practicing the present invention with various indirectly labeled probes;

Fig. 6 is a plot of probe concentration versus probes per cell at a given number of cells present with the probe concentration in ng/ml being shown as abscissae, and the number of probes per cell being shown as ordinates;

Fig. 7 is a plot of hybridization time versus number of probes per cell at a given number of cells present with hybridization time in minutes being shown as abscissae, and number of probes per cell being shown as ordinates;

Figs. 8 and 9 show plots illustrating the effect of probe size in relation to the level of fluorescence obtained using, in one plot an 18 base pair universal probe (Fig. 8), and, in another plot, a 35 base pair specific probe (Fig. 9); where, in both plots, concentration is shown in molecules per ml as abscissae for each of 30 min., 60 min. and 90 min hybridization times, and relative fluorescence is shown in instrument units as ordinates;

Fig. 10 is a graph illustrating assay sensitivity by fluorescence quantitation with E. coli cells per assay being shown as abscissae, and relative fluorescence in instrument units being shown as ordinates;

Fig. 11 is a graph illustrating specificity of in situ hybridization for each of a universal probe and an E. coli specific probe tested on each of E. coli and Proteus cells with hybridization time in minutes being shown as abscissae, and relative fluorescence in instrument units being shown as ordinates;

Fig. 12 is a graph illustrating the effect of cell growth rate on hybridization efficiency using a specific organism species and a universal probe with elapsed incubation time in minutes being shown as abscissae, and relative fluorescence and also relative cell density, each in percent, being shown as ordinates;

Fig. 13 is a graph illustrating the relationship between the total amount of methanol and formaldehyde in the mixture (in µl) per one ml of aqueous treating medium shown as abscissae against absorbance at 260 nm as a measure of cell density shown as ordinates; and

Fig. 14 is a graph illustrating the relationship between total amount of methanol and formaldehyde in the mixture (in µl) per one ml of aqueous treating medium shown as abscissae against photon emission per hybridized sample shown as ordinates.

## Detailed Description

### (a) Starting Materials

Microorganisms employed in the practice of this invention are single cells of microbial origin. Suitable microorganisms for use as starting materials in the practice of this invention are now believed to comprise, in general, living organisms of microscopic size, including bacteria, yeasts, molds, fungi and the like whether or not such are infected with viruses or bacteriophages. Exact cell size is relatively unimportant. If the bacteria can sporulate, then it is preferred that they be in the vegetative (non-sporulative) state.

Preferred microorganisms suitable for use as starting materials in the practice of this invention are characterized by having a cell wall which normally provides a probe permeability barrier. In nature, such a cell wall protects the microorganism from a wide variety of antimetabolites (such as actinomycin), detergents, drugs, and enzymes (such as lysozyme). Examples of such microorganisms include bacteria and yeasts which are presently more preferred as starting microorganisms for use in the practice of this invention. Particularly preferred starting microorganisms are prokaryotes.

Microorganism single cells for use in the practice of this invention can be obtained from any convenient or desired source. For example, cells may be preliminarily harvested from broth cultures by centrifugation, or the like. Also, cells may be taken directly from culture plates, or from natural sources, such as water, food stuffs, clinical samples, or the like.

Typically, starting microorganism cell concentrations of about $10^9$ cells per ml are convenient. Presently preferred starting cell concentrations for contacting with a treating solution (as described herein) are in the range of about $10^7$ to about $10^9$ cells per ml, but larger and smaller concentrations can be used, if desired.

A suitable treating composition for purposes of this invention contains a combination of a conventional fixing agent and a conventional lipid solvent.

In accord with the present invention, it has now been discovered that, when such a starting microorganism is contacted under liquid phase conditions with such an aqueous treating composition of fixing agent and lipid solvent, such microorganism becomes fixed and also its cell wall becomes permeabilized to an extent such that the cell wall thereof can be permeated (that is, traversed) readily and quickly under aqueous liquid phase conditions by an oligonucleotide.

The term "fix", "fixing", "fixes", "fixation", or like word form, as used herein, refers to stabilizing or the stabilization of a microorganism substantially in its starting (or natural) state for purposes of practicing the present invention.

Such oligonucleotide preferably contains about 18 to about 40 nucleotides per molecule, and such preferably permeates the cell wall within a time of not more than about 60 minutes at 37° C, at a cell concentration of about $10^9$ per ml, and at an oligonucleotide concentration in the range of about $4 \times 10^{14}$ to about $8 \times 10^{14}$ oligonucleotide molecules per ml, in an aqueous medium under conditions which will support or promote hybridization.

Additionally, the effect of contacting the composition of fixing agent and lipid solvent with the microorganism is such that the cellular morphology of the resulting microorganism is not appreciably altered, as shown by visual examination thereof with a light microscope at a magnification of, for example, about 1000x.

In addition, the effect of such contacting is such that the resulting microorganism is centrifugable without appreciable alteration in cellular morphology (similarly determined) and without substantial cell population loss. Centrifuging is desirable and presently preferred to separate the microorganism from an aqueous medium. Such a centrifuging is typically conducted with a force in the range of about 13,000 to about 16,000 g applied for a time sufficient to effect such a separation which time is typically less than about 10 minutes.

The capacity of an oligonucleotide to permeate, that is, traverse and enter, into the cell interior of such a fixed and cell wall permeabilized cell can be measured, if desired, by any convenient means. To evaluate such capacity, for example, an oligonucleotide probe can be exposed to the cell under aqueous phase conditions. Probe hybridization is not necessary; only the probe label is subsequently employed in such a

permeation test for the purpose of establishing upon subsequent detection that the oligonucleotide has entered the cell. For another example, an unlabeled oligonucleotide, such as one contained in a single stranded primary probe, can be similarly exposed to the cell. Then, a labeled, single-stranded secondary probe that does not contain a complement to a target sequence, but which contains an oligonucleotide sequence that is complementary to the primary probe, is contacted with the treated cell, allowed to traverse the cell wall, enter the cell, and hybridize to the unlabeled oligonucleotide probe, thereby labeling the whole hybrid complex in the cell for subsequent detection to show that the probe has entered the cell; see, for example, European Pat. Application No. 0079139.

In such a contacting of such a treating composition with starting cells, as those skilled in the art will appreciate, various fixing agents known to the art can be employed, such as formaldehyde, paraformaldehyde, glutaraldehyde, or other aldehyde, or the like; and various lipid solvents can be employed, such as methanol, ethanol, or other alkanol, or the like. Formaldehyde and methanol are particularly preferred. Such a combination is preferably miscible (soluble) in water and is preferably employed in an aqueous treating medium wherein the quantity of water is preferably in the range of about 20 to about 34 volume percent with the total volume percent of such combination of fixing agent and lipid solvent comprising about 66 to about 80 volume percent of a given such treating composition on a 100 volume percent total liquid treating composition basis. The relative respective quantities and identities of the fixing agent and of the lipid solvent employed can vary. For example, the amount of an aldehyde fixing agent can be in the range of about 1 to about 5 volume percent while the amount of a lipid solvent can be in the range of about 65 to about 75 volume percent on a 100 volume percent total treating composition basis.

One particularly effective and presently most preferred starting cell treating composition, which can itself be used, if desired, for purposes of screening a given starting microorganism to establish suitability or desirability of using such given microorganism in the practice of this invention, comprises a mixture of about 72 volume percent methanol, about 3.6 volume percent formaldehyde and about 24.4 volume percent water on a 100 volume percent total liquid basis.

The effect of cell treatment with an aqueous methanol-formaldehyde mixture is illustrated, for example, in Figs. 13 and 14. Thus, Fig. 13 shows the recovery of cells by centrifugation at the end of a diagnostic procedure (including hybridization) that is in accord with the present invention wherein, for example, fluorophore labeled probes are used. Fig. 14 shows the amount of fluorescence (in number of photons) obtained from a sample that is processed in accord with the present invention using fluorophore labeled probes. The curve described by the solid squares is the absolute fluorescence, which becomes less with increasing methanol-formaldehyde concentration due to cell loss. Once these values are corrected for the cell loss that occurs in centrifugation, the optimum condition appears to be near the presently most preferred treating composition above indicated (i.e., 800 $\mu$l of methanol-formamide mixture per 1000 $\mu$l of an aqueous reaction mix, which translates into 72 volume percent methanol, and 3.6 volume percent formaldehyde).

These data of Figs. 13 and 14 suggest that a wide range of methanol-formaldehyde concentrations (0 to about 90% methanol and 0 to about 3.7% formaldehyde) are suitable for use in the practice of this invention with an optimum being a mixture of methanol and formaldehyde in water wherein the respective amounts of each is near the value hereinabove indicated for a most preferred treating composition. By keeping the cells under evaluation in an aqueous dispersion, a cell fixing and cell wall permeabilizing step is less important and may not even be necessary (see the "0" point in Fig. 14). This has profound consequences, as the cells can then stay alive during hybridization and detection.

For example, in the case of certain microorganisms, probes can be added to an aqueous dispersion thereof so as to effectuate hybridization in the microorganism cell even when such microorganisms are not first treated with a treating composition of fixing agent and lipid solvent as described herein. Thereafter, detection can be carried out as taught herein.

In each of Figs. 13 and 14 the location A in each instance designates a presently most preferred condition while the location B in each instance designates the treating condition employed in the prior art with slide mounted cells (Pace et al., above cited and discussed).

An aqueous composition, such as is used for suspending (i.e., dispersing) microorganisms undergoing hybridization in accord with the teachings of this invention, should preferably contain dissolved therein additives, such as salts, buffering agents, and the like, which tend to permit or promote hybridization of a probe to the cellular target sequence. In addition, such additives may stabilize the suspended microbial cell and prevent undesired morphological changes therein. Such additives and the respective effective amounts of each are known to the art. Examples include ethylene diamine tetraacetic acid (EDTA), borates, TRIS (including TRIS-HCl), and the like. For example, a suitable such aqueous medium may contain a mixture of the following solutes:

**TABLE I**

**EXEMPLARY HYBRIDIZATION MEDIUM**

| | |
|---|---|
| 4.5 mM | alkali metal borates (sodium preferred) |
| 675 mM | alkali metal chlorides (sodium preferred) |
| 4.5 mM | ethylene diamine tetraacetic acid (EDTA) |
| 90 mM | TRIS-HCl (pH 7.8) |
| 9 wt % | dextran (final comp.) |
| 0.18 wt % | bovine serum albumin (BSA) (final comp.) |
| 0.009 wt % | polyadenosine (final comp.) |

If cell washing is to be carried out prior to, or after, hybridization as taught herein, a buffered aqueous medium, such as one which is not pH regulated with TRIS-HCl, can be used, if desired.

In addition to its usefulness as a screening agent, the above indicated presently most preferred treating composition of methanol, formaldehyde and water is also useful for purposes of standardizing a diagnostic procedure that is based upon the teachings of the present invention, as those skilled in the art will readily appreciate. For example, uniform treatment of a microorganism culture with such a treating composition is desirable in order to practice an embodiment of a method of this invention. A standardized procedure is replicatable anywhere in the world as is appreciated in the art, and can be used for any purpose within the scope of this invention, such as, for example, the identification or characterization of a single species of bacteria in a mixed culture, or the like.

While various treatment procedures for fixation of cells and tissues have been described in the prior art, a specific treatment procedure for the purposes of fixation and cell wall permeabilization as taught herein for use in in situ hybridization in aqueous suspension for single cell microorganism evaluation or diagnosis is not believed to have been heretofore employed in the art; see, for example, the discussion and references cited in Singer et al. U.S. Pat. No. 4,888,278 (although the in situ hybridization teachings of Singer et al. are obviously distinctly different from the present invention).

Probes suitable for, or selected for, use in the practice of the present invention can be those known to, and made by, prior art teachings. However, in in situ hybridization as practiced by this invention, it is presently preferred to employ probes comprised of at least one oligonucleotide and at least one label moiety per probe molecule. The oligonucleotide portion used for hybridization in the practice of this invention can contain about 8 to about 100 nucleotides per molecule, but it is presently preferred to employ oligonucleotides which contain about 18 to about 40 nucleotides per probe molecule. The individual nucleotides are chosen and arranged relative to one another in the oligonucleotide so as to provide a nucleotide sequence that is complementary to at least one nucleotide sequence found in at least one nucleic acid component present in a microorganism being evaluated or diagnosed in accord with the present invention. The oligonucleotide sequence can be targeted for complementary nucleotide sequences in a cellular nucleic acid so that the probe can be considered to be either a DNA probe or an RNA probe as the case may be. Oligonucleotides in such a size range appear to be desirable not only because such a size appears to be generally sufficient for identification of a particular nucleotide grouping in a single celled microorganism for most purposes, including gene identification, or the like, but also because the relatively small size associated therewith evidently enhances the capacity of such a probe to traverse a microorganism's permeabilized cell wall and to move through intracellular material on its way to coupling with a complementary cellular nucleotide sequence for hybridization.

Likewise, and similarly, it is also preferred that the label moiety be chemically associated with a probe and be relatively small in volumetric size. From evidence now available, it does not appear that the presence of more than one label moiety per probe molecule appreciably alters the in situ hybridization capacity of a probe for purposes of practicing the present invention. Therefore, the presence of two or more label moieties per probe molecule is a present preference particularly when low copy number cellular nucleic acids are targeted. Multiple label moieties per probe molecule appear to enhance the detectability of hybrids formed therewith in a microorganism structure in accord with the present invention. Usually a probe does not contain more than 5 label moieties per probe molecule. Usually not more than about 5% of a probe molecule's total oligonucleotide contains label moieties.

Methods for the preparation of suitable nucleic acid probes for use in the practice of this invention are provided in the prior art; see, for example, the J.E. Arrand article "Preparation of Nucleic Acid Probes" in

the aforementioned book "Nucleic Acid Hybridization" at pp 17-44.

In general, and as those skilled in the art will appreciate, the choice of probe is mainly influenced by the target nucleotide sequence contemplated and by the technique of detection contemplated. The fact that hybridization is occurring in a cell structure in accord with this invention does not appear to limit the type of labeling employed in a probe. Thus, probe labels and labeling detection procedures known to the prior art generally can be employed in this invention. Presently preferred probe labels include fluorophore-containing moieties, radioisotope-containing moieties, and enzymatically active moieties.

(b) Process Conditions and Products

As above indicated, in a first procedural step of a cell diagnostic procedure of this invention, whether or not one is testing to determine the possible presence of a microorganism, such as a given microorganism species, phylogenetic group, class or the like, or whether or not one is testing to determine the approximate quantity of such a microorganism that is already known to be present, and assuming for present discussion purposes that such a microorganism is present, one contacts such microorganism in an aqueous medium with a combination of fixing agent and lipid solvent such as above indicated. Such treatment fixes the microorganisms and permeabilizes their cell walls, as described above.

To preserve the structural integrity of the microorganism undergoing test, and thereby maximize test accuracy and reliability, the aqueous medium wherein the microorganism is dispersed should preferably also have characteristics and dissolved components therein such as are above indicated herein. Although the contacting temperature in this step can vary widely, it is presently preferred to employ a treating composition contacting temperature in the range of about 15 to about 37° C. Contacting times can suitably and preferably be in the range of about 5 to about 30 minutes. Higher and lower contacting temperatures and/or times can be used, if desired. Contacting times appear to be relatively unimportant so far as effect upon the preferentially employed and so treated microorganisms is concerned. Thus, although contacting times of up to about 2 hours are feasible, such are generally not necessary.

The concentration or cell density per unit volume of aqueous treating medium of microorganism population being so contacted with treating composition appears to be relatively unimportant so far as individual microorganism fixing and cell wall permeabilizing is concerned. A suitable, convenient and illustrative balance for starting cell density is in the range of about $10^4$ to about $10^6$ cells per µl (equivalent to about $10^7$ to about $10^9$ cells per ml) of treating medium. Larger and smaller such densities, of course, can be used, if desired or necessary because of circumstances.

It is greatly preferred to separate a treating composition from the microorganisms treated therewith, and then to resuspend the separated treated microorganisms in a fresh aqueous medium, before such treated microorganisms are contacted with probes. The reason is that the treating composition may interfere with, or even prevent, the desired hybridization from taking place. For example, the methanol in the treating composition at the relatively high concentrations now presently preferred (e.g. 72 volume percent, see above) could prevent formation of the desired probe target hybrid complex.

The so treated cells are thus preferably separated from the aqueous treating medium by centrifuging or the like and then thereafter resuspended in a fresh aqueous probe treating medium whose characteristics are as hereinabove indicated. Suitable centrifuging conditions can be as above stated; thus, centrifuging at a force in the range of about 12,000 to about 18,000 g for a time of less than about 10 minutes is generally sufficient to effect a cell separation from a treating composition.

It is generally preferred but is not necessary to wash recovered cells before resuspending. The cell suspending medium itself can be used for washing. Washing can conveniently be accomplished by resuspending the cells in the suspending medium and then effectuating another centrifugal cell separation, such as above described. Any convenient method for practicing a separation, optional washing, and resuspension procedure can be employed in practicing this invention.

Either during or after the resuspension, in an aqueous probe treating medium such as above indicated, one contacts the resulting so treated microorganism cells with at least one nucleic acid hybridization probe such as herein above characterized. Contacting is conveniently accomplished by simple mixing. At the time of such probe contacting with treated cells, it is desirable, but not necessary, that the microorganism density in the suspending liquid phase be known. The detection method employed may be influenced by the concentration of cells to be evaluated, as those skilled in the art will appreciate. During such contacting between probes and cells, it is preferred to agitate gently the aqueous probe/cell mixture. The probe/cell contacting temperature is preferably in the range of about 15 to about 37° C, but higher and lower contacting temperatures may be employed, if desired. Contacting time between probe molecules and cells in the aqueous probe contacting medium can vary, but a preferred contacting time lies in the range of about

15 minutes to about 60 minutes. A presently more preferred contacting time is in the range of about 20 to about 30 minutes before proceeding to the next major diagnostic step. Longer contacting times, such as times up to about 2 hours or the like, may actually tend to reduce the number of labeled hybrids available for detection for presently unknown reasons.

While the respective concentrations of treated cells and probes in the aqueous probe contacting medium can vary greatly, typical and illustrative probe concentrations are in the range of about $10^{14}$ to about $10^{15}$ probe molecules per ml at the start of probe/cell contacting, and typical and illustrative cell concentrations are in the range of about $10^7$ to about $10^9$ cells per ml. A typical and convenient ratio of total probes to total cells during probe contacting in an aqueous probe contacting medium lies in the range of about $10^7$ probes per cell to about $10^5$ probes per cell, although larger and smaller such ratios can be employed, if desired.

Various modifications of the probe/cell contacting procedure are possible and can be preferred, depending upon the objectives of this procedure at the time of use. Principle variables include type of probes and microorganisms undergoing evaluation, cellular nucleic acid target(s), method of label detection contemplated, microorganisms to be identified or quantified, and the like, as those skilled in the art will readily appreciate. Some illustrative modifications are hereinbelow provided.

After such a probe/cell contacting procedure, and when time is of the essence, one can proceed directly to the next major processing step which is detection. In detection, the presence of labels associated with cellular hybridized nucleic acid components in individual so contacted microorganisms is detected. As indicated, a convenient detection procedure for use in the practice of the present invention can be selected from among the various known prior art detection procedures. Exemplary detection procedures are hereinbelow described.

At the end of the probe/cell contacting, the concentration of probe molecules in the aqueous probe contacting medium relative to their concentration in hybridized individual cells in the same medium is characteristically and preferably usually relatively low. For example, in the illustrative typical situation above described, there are typically less than about 100 extra-cellular probe molecules in the volume of the aqueous medium that is occupied by a single cell. Thus, a separation of such liquid medium with its residual probes from resulting so contacted cells may not be necessary before probe label detection is carried out, depending on the detection procedure being used. The need for such a separation is thus mainly influenced by the particular detection procedure contemplated, as hereinbelow illustrated.

In general, time permitting, for maximum accuracy of probe detection, it is preferred after the probe/cell contacting and before the detection, to separate the resulting dells from the extra-cellular residual probes remaining dissolved in the aqueous liquid medium phase by centrifuging the resulting mixture. Removing unhybridized probes from regions exterior to and around resulting probe contacted microorganisms is desirable before commencing detection because the unhybridized probes, with their labels, can give rise to a false level of detection in relation to the actual hybrids existing in the microorganism(s) undergoing evaluation, depending upon the detection method, as those skilled in the art will appreciate.

Any convenient complete or partial separation procedure, subsequent washing procedure, and subsequent resuspending procedure (if desired) can be employed. Although, for example, after such centrifuging, detection can be carried out on the recovered cell mass directly, it is presently preferred thereafter to resuspend the recovered cells in a fresh aqueous treating medium whose characteristics are as hereinabove indicated for an aqueous probe treating medium before proceeding to the diagnostic step (the detection procedure).

If the cells are separated before detection, then cell flushing is preferably carried out. The flushing procedure can be as above described. Preferably, the flushing medium is aqueous and contains additives which stabilize the label moiety, as is known to the art, such as borated buffers in the case of fluorophore labels, and the like.

Before the first procedural step (above indicated) of a cell diagnostic procedure of this invention is undertaken, one can pretreat the starting living microorganism cells to increase therein the copy number of the particular cellular nucleic acid components that are being targeted for probe hybridization. For one thing, when the cells undergoing such a procedure are in an actively growing state, the number of ribosomal RNA molecules per cell is heightened. In the case of bacteria, 16S rRNA molecules in such a growing state can be present in up to about 100,000 copies per cell, as taught by Pace et al. (references cited above). All of such copies are available for hybridization under the process conditions employed in the present invention, as shown by the Examples below.

To achieve, for example, a maximized number of 16S rRNA molecules per cell, the starting living microorganism can be cultured (that is, subjected to conditions which promote microorganism growth) just before undergoing such a diagnostic procedure that is in accord with the present invention. Optimal growth

media for the various different microorganism species are known to the art.

Procedures already known to the art other than those involving cell growth or cell culture can be used preliminarily to pretreat starting microorganisms and to enhance the copy number of cellular nucleic acid target sequences. For example, the increase in the number of certain plasmids is achieved by cell culture in the presence of certain antibiotics.

Either immediately before or during probe contacting with suspended cells, that is, after contact with treating composition and before the end of treated cell contacting with probes, the microorganism cells (and the associated suspending medium) can be quickly heated to a temperature in the range of about 80 to about 100°C (about 90 to about 95°C being presently preferred) in order to promote the denaturation of the normally double stranded cellular target nucleic acid molecules and thereby enhance the capacity to such molecules to hybridize with probes. The duration of such heating extends for about 1 to about 5 minutes followed thereafter by a quick temperature reduction to about 4°C or the like. Longer and shorter times may be used if desired. Subsequently, the resulting cell temperature is raised to the desired hybridization temperature which, as above indicated, is preferably in the range of about 15 to about 37°C (although higher and lower such temperatures can be used, if desired).

Because of the inherently large number of possible starting microorganism samples which can be used in the practice of this invention, it is not possible to define a single exact processing procedure that is universally applicable to all practices of this invention. One presently preferred and illustrative mode of practicing the process of this invention involves the use of a microcentrifuge, such as an Eppendorf, or the like. For example, using a sample having a cell concentration within the starting range above illustrated, one could employ from about 1 to about 5 ml of a starting aqueous suspension sample and then process such sample using the following illustrative and presently preferred procedural step sequence:

(a) Centrifuging the starting cell sample preferably using centrifuging conditions in the range above described and then resuspending the recovered cells in an aqueous medium which is conveniently about 1/10 the volume of the liquid in the starting cell sample although a greater or smaller volume of such medium may be employed if desired. Such aqueous medium is preferably one such as is described herein which contains about 5mM borate and which does not have an adjusted pH. Such medium acts as a wash.

(b) Recentrifuging such cell sample preferably using centrifuging conditions in the range above described and then resuspending the recovered cells in a fixing and cell wall permeabilizing treating composition using a treating composition volume which conveniently is about 1/10 of the volume of the liquid of the starting sample. Such treating composition is preferably a mixture of methanol, formaldehyde, and water, such as above described. The contacting time is preferably about 10 minutes and the contacting temperature is conveniently room temperature (preferably about 18 to about 25°C) with mild agitation.

(c) Recentrifuging the resulting cell sample preferably using centrifuging conditions in the range above described and then resuspending the recovered cells in an aqueous medium which is conveniently about 1/5 to about 1/10th the volume of the liquid in the starting cell sample. Such aqueous medium is likewise preferably one such as described herein which contains about 5 mM borate and which does not have an adjusted pH. Such medium acts as a wash, as before.

(d) Recentrifuging the resulting cell sample preferably using centrifuging conditions in the range above described and then resuspending the recovered cells in an aqueous medium suitable for hybridization, preferably one such as is described herein containing the TRIS-HCl and having an adjusted pH. The volume of such medium is preferably about 100 to about 300 µl. Dissolved in this aqueous medium are fluorophore-labeled oligonucleotide probes that are targeted for specific cellular nucleotide sequence(s). A suitable probe concentration is about 0.75µg/ml, but larger and smaller such concentrations can be used, if desired, as taught herein. The probe/cell contacting time is preferably about 20 to about 30 minutes at a temperature in the range of about 35°C to about 65°C with continuous agitation (preferably about 37°C to about 40°C with mild agitation).

(e) Recentrifuging the resulting probe-contacted cells preferably using centrifuging conditions in the range above described and then resuspending the recovered cells in an aqueous medium which is conveniently about 1/5 to about 1/10th the volume of the liquid in the starting cell sample. Such medium is preferably one such as above described which contains borate and has no adjusted pH. Such medium acts as a wash.

(f) Recentrifuging the resulting cells preferably using centrifuging conditions in the range above described and then resuspending the recovered cells in an aqueous medium whose volume is preferably selected so as to be suited for the particular detection procedure to be subsequently employed. For example, typically, such volume is influenced by the size of the cuvette employed in the case of

fluorescence detection. Typically such volume falls in the range of about 1/20th up to about the original volume of the starting sample. Such medium is preferably one such as above described which contains the borate and has no adjusted pH.

(g) Detecting fluorescence of the resulting cells. For example, the cells can be directly observed with a fluorescence microscope. Alternatively, and for another example, the cells can be passed through a fluorometer to measure their fluorescence at particular photon emissions associated with the fluorescence characteristics of the fluorophores employed in the probes. Alternatively, and for another example, a flow cytometer can be used.

Practice of this invention is better understood by reference to the flow diagrams shown in the attached drawings (see Figs. 1-5):

Referring to Fig. 1, there is seen a flow diagram for a simple embodiment of the present invention which is capable of being rapidly carried out. In a first step, the microbial single cells undergoing evaluation are treated while suspended in a liquid phase with a treating composition comprising a cell fixing agent and a lipid solvent to achieve permeabilization of cell walls. The resulting cells are then separated and resuspended in fresh aqueous medium. In a next successive step, the so treated cells are mixed with labeled nucleic acid probes to allow hybridization of targeted cellular nucleic acid. In a final step, the resulting cells are subjected to probe detection and evaluation. Adjustment of procedural parameters permits execution of these steps in a very short time interval and achievement of accurate results.

Referring to Fig. 2, there is seen a presently preferred embodiment of a process step sequence for practicing this invention. Here, the starting living single cell microorganisms to be evaluated are preliminarily treated as above explained to enhance therein the nucleic acid target material(s) being pursued by the probe(s) employed, as herein above explained. Thereafter, in a first diagnostic procedural step, the cells are contacted with a liquid treating composition which fixes the cells and permeabilizes their cell walls, as taught herein. Thereafter, the so treated cells are separated from the liquid treating composition preferably by centrifuging and resuspended. In a next step, labeled nucleic acid probes are mixed and contacted with the so treated cells under the aqueous phase conditions to allow hybridization to occur, as taught herein. When, for example, cellular ribosomal RNA is targeted to be hybridized, probes are used which allow hybridization to the rRNA targeted. When, for another example, DNA is to be hybridized, probes are used which allow hybridization to a targeted cellular DNA nucleotide sequence. Residual or remaining extracellular (unhybridized) probes are optionally but preferably separated, preferably by centrifugation, and the hybridized cells are preferably resuspended in an aqueous medium, as taught herein. Finally, the resulting cells are evaluated by probe label analysis.

Application of the step sequences of the Fig. 1 and Fig. 2 process embodiments to a microorganism evaluation procedure wherein microorganism cells are evaluated using fluorescently labeled (fluorophore labeled) probes is exemplified in Fig. 3. Starting cells are primarily treated as described in Figs 1 and 2 and then separated and resuspended. Then, after fluorophore labeled probes are mixed with the treated cells, and after an opportunity for hybridization has occurred, either (1) microscopy or image analysis is first directly undertaken, or, alternatively, (2) extra cellular unhybridized probes are first separated from the residual probes, and thereafter fluorometry, capillary electrophoresis combined with laser-enhanced fluorescence detection, FACS (fluorescent activated cell sorter) analysis, or the like, is carried out. Various alternative procedural pathways are indicated with arrows in this Fig. 3.

In microscopic evaluation of fluorescence, a representative sample of the aqueous cellular dispersion which has been subjected to hybridization is placed under microscopic examination in a fluorescence microscope and individual cells are observed to determine whether or not hybrid formation has occurred therein as shown by cell fluorescence.

In image analysis, a representative sample of the aqueous cellular dispersion which has been subjected to hybridization is placed under a microscope that is equipped with a camera and images are subsequently examined by a human observer or by a computer (preferred) to determine cell number and/or fluorescence intensity.

In capillary electrophoresis and laser enhanced fluorescence detection, an aqueous cellular dispersion which has been subjected to hybridization is placed adjacent a capillary network and subjected to an electric field. The cells migrate through the capillaries and pass by a window where they are excited by a laser beam. The emitted fluorescent light is detected through an objective lens system and quantitated. This device is very sensitive and allows readily the detection of a single cell in a sample produced by the practice of this invention. Also, this device lends itself to automation. While capillary electrophoresis instruments are commercially available from a variety of vendors (such as Beckman Instruments), laser enhanced detection systems currently are assembled on site from commercially available subcomponents.

In fluorometry, an aqueous cellular dispersion which has been subjected to hybridization and from

which the unhybridized probes have been removed is placed into a cuvette and then subjected to measurement of total fluorescence with a fluorometer. A suitable fluorometer is available commercially from SLM Instruments, Inc. under the designation "SPF-500 spectro fluorometer".

In FACS analysis, an aqueous cellular dispersion which has been subjected to hybridization and from which unhybridized probes have been removed is passed in an aqueous dispersion through a fluorescent activated cell sorter and the number of signals detected is counted. Such a fluorescent activated cell sorter is available commercially under this trade name "Epics 752 Flow Cytometer" from the Coulter Electronics Company.

Application of the step sequences shown in the process embodiments of Figs. 1 and 2 to microorganism evaluation using probes labeled with radioisotopes is illustrated in Fig. 4. Here, fixed and permeabilized cells are separated, resuspended and contacted with radioisotope labeled probes. After an opportunity for hybridization has occurred, then either (1) filtration, radioisotope detection, or the like, is carried out, such as by slot blotting, autoradiography, or the like, or (2) extracellular unhybridized probes are first separated from treated cells, and then are subjected to scintillation counting, or the like, with the hybridized cells being dispersed in an appropriate aqueous medium such as described.

In filtration detection, an aqueous dispersion of product cells is filtered through a conventional filtration device, such as a slot blotter, or the like, and then are subjected to quantitation of radioactivity by autoradiography, instrument aided radiation quantification, or the like.

In scintillation counting, an aqueous dispersion of product cells is placed into a scintillation counter and the amount of radioactivity detected is quantitated. Various suitable such counters are available commercially.

Application of the step sequence of the embodiments of Figs 1 and 2 to microorganism evaluation using probes which are indirectly labeled is illustrated in Fig. 5. Here, after preliminary cell fixing and permeabilizing, and then separating and resuspending, the appropriately indirectly labeled probes are mixed with treated cells allowing probe penetration of cells and hybridization to occur.

If, for example, the probes are labeled with biotin or hapten, then, after hybridization has occurred, unhybridized probes are separated and the resuspended hybridized cells (preferably in an aqueous/medium such as hereinabove described) are admixed under liquid phase conditions with a coupling agent, such as a labeled material. Such material can be, for example, selected from the group consisting of avidin, streptavidin, or antibody. The label moiety of the labeled material can be, for example, conveniently selected from the group consisting of fluorophore-containing, radioisotope-containing, and chromogen- or enzyme-containing label moieties. More than one such label moiety per probe molecule can be present. Thereafter, the excess or unbound (and extracellular) labeled material is separated by centrifuging. The resulting cellular material is resuspended in an aqueous medium. Thereafter, detection is accomplished with the particular evaluation procedure used being dependent upon the label moieties used.

If the probes are labeled with an enzyme, then, after hybridization has occurred, unhybridized probes are separated, recovered cells are resuspended (preferably in an aqueous medium as above described), and the resuspended hybridized cells are admixed under liquid phase conditions with an identifying agent which comprises, for example an enzyme converted substrate that is a chemiluminescent precursor moiety. The enzyme converts this moiety into a chemiluminescent agent which emits photons. Alternatively, a chromogen substrate is added which converts by enzyme action into a colored body. Optionally, the excess, or unbound and extracellular, coupling agent is separated by centrifuging using, for example, conditions, such as hereinabove described. The resulting cellular material is resuspended in an aqueous medium preferably, such as hereinabove described. The photons generated are evaluated by microscopy, image analysis or luminometry. Detection is accomplished with the evaluation procedure being dependent upon the label moieties used.

In microscopic evaluation of chemiluminescence, a cell sample in an aqueous dispersion is placed under a light microscope and observed directly without incident light from the microscope.

In image analysis of chemiluminescence, cells in an aqueous dispersion are placed under a microscope equipped with a camera and the cell number and/or luminescence is determined by a human operator or by a computer (preferred).

In luminometry, cells in aqueous dispersion are placed into a luminometer and the total amount of luminescence is determined.

If the probes are labeled with avidin, streptavidin, or antibody, then, after hybridization has occurred, unhybridized probes are separated, the recovered cells are resuspended (preferably in an aqueous medium as above described), and then the resuspended hybridized cells are admixed under liquid phase conditions with an identifying agent which contains one or more label moieties. The label moiety can be conveniently selected from the group consisting singly or in combination of fluorophores, radioisotopes, chromogens,

enzymatically active proteins attached to biotin or haptens, or the like. The excess or unbound (and extracellular) identifying agent, such as the unbound biotin or hapten, is separated by centrifuging. The resulting cellular material is then resuspended in an aqueous medium as hereinabove described. Thereafter, detection is accomplished with the evaluation procedures being dependent upon the label moieties used.

The present invention makes possible a significant variety of new and very useful diagnostic procedures for single cell microorganism identification and/or characterization. Exemplary such procedures are:

1. Contacting a preliminary fixed and permeabilized microorganism population with at least two different probes simultaneously. One such probe incorporates an oligonucleotide having a nucleotide sequence which is of the universal type; that is, the sequence is complementary to a particular nucleotide sequence that is common to all known microorganisms of a defined kind. For instance, a nucleotide sequence can be used which is common to the 16S ribosomal RNA as hereinbelow exemplified. Such probe is labeled with a distinctive label, such as a fluorophore of one fluorescing color. Another such probe incorporates an oligonucleotide having a nucleotide sequence which is species specific; that is, the sequence is complementary to a particular nucleotide sequence that is found only in one bacterial species, such as E. coli, as hereinbelow exemplified. Such probe is labeled with a distinctive label, such as a fluorophore of one fluorescing color which is different from the first above color. In place of the universal probe, a probe common to two or more bacterial species can be used, if desired. Such a probe mixture makes practical the identification of all microorganisms present in a specimen which are identifiable by the chosen universal probe as well as the identification of any microorganisms present which are identifiable by the species specific probe. One significant feature of this procedure is that it can utilize to a maximum extent the circumstance that the only stable cellular nucleic acid target with a high copy number per cell is rRNA. Hence, rRNA, which can be present in quantities up to about 100,000 copies per cell, can be used to provide a source of nucleic acid for producing the above indicated required 10,000 fluorophores per cell using oligomeric DNA probes directly labeled with one fluorophore per probe molecule for fluorescent detection. Messenger RNA is generally present in lower copy number (1000 molecules or less since it is either very unstable or is produced in such a lower copy number). Plasmid DNA molecules can be present with up to 1000 copies, while the bacterial chromosome is present in only one copy. However, there are no sequences on the chromosomal or plasmid DNA that are repeated to anywhere near the number that one could bind 10,000 probes (of a unique sequence).

As one example, one can consider the microbiological analysis of stool samples from patients with diarrheal disease. The typical intestinal flora of a healthy human contains between $10^{12}$ and $10^{14}$ bacterial cells of many different classes per gram of feces, while the diarrhea-causing pathogenic organisms may be present in concentrations several logs below that figure. Thus, in the currently standard identification process, the causative organism has to be isolated away from the normal intestinal flora before it can be identified. The total time requirement for this procedure is typically 3 days. Since the bacterial diarrheas are typically caused by one of only a few bacterial species (enteropathogenic E. coli, Salmonella, Shigella, Vibrio, Aeromonas, or Campylobacter), the presence of one of these organisms is demonstrable without prior isolation by application of the present invention. For that purpose, an aliquot of the fecal sample can be treated as detailed in Fig. 2 for hybridization to a panel of fluorescently tagged probes in a 2 to 3 hour procedure. For an example, if this panel were to include a universal probe labeled with T-RED and a Salmonella specific probe labelled with FITC, all cells are labeled RED (observed under the fluorescent microscope or measured at the appropriate emission wavelength with the fluorometer), while only Salmonella cells are detectable when observed under the microscope with the blue filter or at the FITC specific emission wavelength with the fluorometer. Using, for an example, 6 different hybridization reactions, one can easily determine the presence of any of the above mentioned pathogenic bacteria in a stool sample.

A modification of the above described situation also serves as an example here. Instead of a mixture of a universal probe and a species specific probe, one can use several different probes simultaneously, each with a different fluorophor, and each specific for one of the pathogenic organisms. By using different filters under the fluorescent microscope, or different excitation and emission wavelengths on the fluorometer, one can detect and identify any of the possible pathogens for which probes are provided in one fecal sample with a single hybridization reaction.

2. Contacting a preliminarily fixed and permeabilized microorganism population with at least two different probes, such as fluorophore labeled oligonucleotide probes. One such probe incorporates a nucleotide sequence which is specific to, for example, one species of bacteria. Another such probe incorporates a nucleotide sequence which is specific to, for example, another species of bacteria. The fluorophores of each probe differ in color from the fluorophores of the other(s) thereof. Thus, hybrids formed with these

different probes in admixture can be separately detected so that different bacterial species present in the same specimen can be detected in a single procedure.

In many instances, identification of a microorganism as belonging to a specific species is not sufficient for its characterization, and additional information is needed. For an example, the typical laboratory identification of pathogenic bacteria includes an antibiotic sensitivity test, because it is not only important to the practicing physician to know what organisms caused the disease, but also to know whether that organism is resistant to certain antibiotics. Other virulence factors, such as the presence of pili, flagella, exoenzymes or toxins are in many cases also independent of the species classification and have to be tested for in addition to the strain identification. The in situ hybridization in suspension technique of this invention allows for both tests, the strain identification test (conveniently and preferably by a specific probe targeted at 16S rRNA), and the test for the special virulence factor, by targeting one or more probes, with or without the herein described amplification, at the gene for this factor or its transcript (mRNA).

As an example, one may consider a test for Corynebacterium diphtheriae, where a throat swab is incubated in a medium that promotes cell growth, but which is devoid of iron, which results in toxin expression. A liquid suspension of cells from this medium is treated according to the present method for in situ hybridization, and then contacted with two different types of probes. One such probe identifies the organisms as being Corynebacterium diphtheriae, and a second probe (or group of probes), specific to the phage-borne diphteria toxin, reveals whether this strain is toxin positive or negative.

3. Contacting the prepared starting microorganisms with at least two different oligonucleotide probes. One of the probes incorporates a nucleotide sequence which is specifically complementary to a nucleotide sequence of the ribosomal RNA in one species of bacteria. A second of the probes incorporates a nucleotide sequence which is specifically complementary to a nucleotide sequence of a DNA found in the same species of bacteria. The DNA can be selected from the group consisting of plasmid DNA and chromosomal DNA. Thus, a single procedure can be used to identify the presence of a given bacterial species and whether or not such species has certain characteristics, such as antibiotic resistance (because of the presence of a particular gene in such DNA), presence of a phage, or otherwise, as desired.

4. Contacting the prepared starting microorganism with at least two different oligonucleotide probes. At least two of the probes are targeted for the same nucleic acid type that exists in a given microorganism. The nucleic acid is selected, for example, from the group consisting of messenger RNA, plasmid DNA and chromosomal DNA. Each of the probes contains a different nucleotide sequence which is complementary to a particular nucleotide sequence located successively and generally longitudinally along adjacent portions of the same target nucleic acid molecule. Thus, during contacting, hybridization of the probes to the same target nucleic acid type occurs. Thus, for example, a significant portion of such molecule is thereby "paintable" with a plurality of fluorophore label moieties so as to generate sufficient signal (photon emission) to make such molecule detectable. By this technique, even individual genes along a chr-DNA can be identified, for example. Thus, the detectability of a target nucleic acid is enhanced.

5. Another such technique likewise involves using under aqueous contacting conditions a plurality (at least two and preferably three) of labeled oligonucleotide probes each incorporating two nucleotide sequences. At least one primary probe has two nucleotide sequences, one of which is complementary to a cellular target nucleic acid nucleotide sequence and the second of which is complementary to a nucleotide sequence that is associated with a secondary probe. Such a secondary probe has two nucleotide sequences, one of which is complementary to the second nucleotide sequence of the primary probe and the second of which is complementary to a nucleotide sequence of a tertiary probe. The tertiary probe can either contain two nucleotide sequences or only a single nucleotide sequence which is complementary to the second sequence of such secondary probe. If the tertiary probe contains two nucleotide sequences, one thereof is complementary to the second sequence of the secondary probe, and the second thereof can be complementary to a fourth probe. A fourth and further probes containing at least two nucleotide sequences each can be used, if desired, each adapted to chain form, as thus indicated, with the preceding and the succeeding probes. Those skilled in the art will readily appreciate that additional and different probes targeted to different sequences of nucleotides may be concurrently used, if desired, in a single hybridization procedure. Usually not more than about six such chain forming probes are employed. Thus, after hybridizing to a cellular target nucleotide sequence, such primary probe becomes successively hybridized to the other related probes in a chain-like manner, thereby accomplishing an amplification effect. Even when only a single label is associated with each of such probes, the result is that the initially hybridized cellular RNA or DNA (present in low copy number)

becomes identifiable and characterizable.

6. By measuring a large number of cells in accord with the teachings of this invention, one can hybridize (and then detect) messenger RNA, or extra-chromosomal DNA targets, or even specific chromosomal targets (genes). The reason is as follows: As the Embodiments below illustrate, one can detect approximately 10,000,000 (Fig. 10) cells by fluorometry or capillary electrophoresis using probes with single label moieties to hybridize with targeted ribosomal RNA. Assuming, for example, that these cells had 50,000 ribosomes, the total number of probes (i.e., fluorophores) required for detection was 5 x $10^{11}$. By targeting a unique gene either on mRNA or on an extracellular DNA, such as a plasmid DNA, (assuming either 20 plasmids or mRNAs per cell) with a probe containing 5 fluorophores, one only needs $5x10^9$ cells for detection. This number of cells is easily obtained from a single isolated microorganism colony. By using 20 different probes simultaneously (i.e., a 1000 nucleotide long sequence broken into 20 pieces of 50 nucleotides each), one can detect specific genes on the bacterial chromosome.

7. Contacting a treated mixed microorganism population with a species specific probe and thereby producing a hybridized microorganism of such species (if present). When this species is present in too low a concentration to be detected by individual cellular microscopic examination, the presence of such hybridized species is reliably accomplished, for example, fluorometrically using an aqueous cell dispersion as taught herein, or by laser enhanced fluorescence, when such is combined, for instance, with capillary electrophoresis.

The usefulness of this application is illustrated in the following example: If a sample contains, for an example, $10^8$ cells/ml of non-target organisms, and only 10 to 100 target organisms in the same sample, this is difficult to detect by microscopy, since typically only 10 to 20 $\mu$l could be placed on a slide and observed in a wet mount. Thus, at best 2 target organisms, and more likely none, are on a slide, which is impossible at worst, and at best very difficult and time consuming to detect. Concentration of this sample 10 or 100 fold makes the total number of organisms too large to view and detect individual cells by microscopy. However, by in situ hybridization in suspension, using fluorescently labeled probes, one can electrophorese part or possibly all of such sample though a capillary of approximately 2 to 100 um diameter, which passes by a laser beam to excite the fluorophores. Appropriate laser and detection systems are available, which allow the detection of 500 fluorophores in a single peak. ("Current Trends in Capillary Electrophoresis", Goodall et al. LC.GC volume 8, pp. 788-799). By using probes directed at rRNA targets, there are at least several thousand fluorophors per cell, thus allowing single organism detection by an automatable procedure. Moreover, the high sensitivity of this technique allows single cell detection even with mRNA, viral RNA, and possibly plasmid, phage and chromosomal DNA targets, if multiple probes ("painting"), multiple fluorophors, and possibly an amplification protocol are employed.

Such processes of this invention are well suited for automated procedures and analysis.

(d) Kits

In a further aspect of the present invention, diagnostic kits are provided which utilize the present inventive teachings.

In one kit embodiment, the rapid detection of bacteria and/or yeast cells is achieved. In addition to an accompanying manual that describes the detection procedure and provides use instructions, this basic kit includes the following components:

- a labeled universal oligonucleotide probe that is complementary to a nucleotide sequence on 16S rRNA which sequence appears to be present in all bacteria and yeasts;
- a labeled microorganism specific probe that is complementary to a nucleotide sequence that is present only in a specific species, subspecies, and/or phylogenetic group of microorganisms;
- all reagents, salts and/or buffers needed for optimal pre-treatment of the microorganism(s) to be diagnosed;
- all reagents needed for post-hybridization treatment (in case of indirectly labeled probes e.g. antibody, streptavidin and the like);
- live control organisms suspended in a glycerol solution, which produce affirmative and/or negative evaluation results when the procedure is practiced, such organisms being lyophilized, or in some other form that preserves viability; and
- reagents that enhance detection by light microscopy, such as counterstains for visualization of the cells, anti-fade agents that reduce the burnout of fluorophores and the like.

In another kit embodiment, the rapid detection of mixtures of microorganisms, or of mixed microorganisms in a microorganism population of medical interest, is achieved. Preferably, such microorganisms are generally found in the same biological niche or are generally present in the same medical sample. For an

example, a "fecal panel" for the rapid detection of human pathogens in stool can contain everything contained in the foregoing basic kit plus:

- a universal probe (rRNA target)
- a specific probe for E. coli strain 0157 (hemorrhagic fever) (DNA or mRNA target)
- a Salmonella specific probe (rRNA target)
- a Shigellae specific probe (DNA or mRNA target)
- a Vibrio specific probe (rRNA target) an Aeromonas specific probe (rRNA target)
- a Campylobacter specific probe (rRNA target)

Each probe in such a kit is present in two configurations. For example, in a configuration (a) each probe is labelled with a red fluorophore, and in a configuration (b) each probe is labelled with a green fluorophore. This allows the simultaneous use of more than one such probe in a hybridization procedure of this invention. Thus, the detection of, for example, Salmonella (stained with a red Salmonella probe) is accomplishable in the presence of all other intestinal bacteria (stained with a green universal probe), or in the presence of E. coli (stained with a green E. coli specific probe). Such procedure utilizes preselected filter sets on a fluorescence microscope, as those skilled in the art of fluorescence microscopy will readily appreciate.

In another kit embodiment, the rapid identification of particular microorganisms and/or specific or special genes is accomplished. For example, the presence of anti-microbial resistance is detected with kits that contain everything in the above described kits plus one or more probes for each of one or more of the specific genes that produce or cause resistance to an antibiotic, or the like.

All such probes in such a kit can be in one of the above mentioned two exemplary configurations. Thus, for example, a probe for detecting the presence of ampicillin resistant E. coli (with a green fluorophore) and probe specific to a beta-lactamase (with a red fluorophore) can be provided. The hybridized cells can be visualized by fluorescence microscopy (or by fluorimetry), and the specific presence of the beta-lactamase hybrid can be detected by fluorimetry by measuring emission in the range of the spectrum characteristic for the red fluorophore.

## Embodiments

The invention is better understood by reference to the following further illustrative embodiments.

Media and buffers: Hybridization buffer was prepared fresh for each experiment as described by DeLong, E.F., Wickham, G.S. and Pace, N.R. (1989) "Phylogenetic stains: Ribosomal RNA-based Probes for the Identification of Single Cells," Science 243: 1360-1363, and contained 0.75 M NaCl, 5 mM EDTA, 100 mM TRIS-HCl, pH 7.8, 10% Dextran, 0.2% bovine serum albumin (BSA), and 0.01% polyadenosine.

The aqueous borate buffer consisted of 5 mM $Na_2B_4O_7$, pH not adjusted, TSB broth that contained 10 g tryptone, and 5 g yeast extract, 5 g NaCl and 3 g KCl per liter of medium. The TSB (Tryptone Soy Broth) broth was purchased from DIFCO and was prepared according to the manufacturer's instructions.

Growth of cells and in situ hybridization: Unless otherwise indicated, all bacterial stock strains were obtained from the Department of Microbiology, East Carolina University School of Medicine. Clinical specimens were obtained from the Microbiology Diagnostics Laboratory, University of Chicago Medical School. Overnight cultures were diluted 100 fold in TYE or TSB and incubation was continued under vigorous shaking at 37°C. Growth was monitored by absorbance at 550 nm.

For diagnosis, all cells were generally harvested at $OD_{550}$ = 0.2 (1 x $10^8$ CFU/ml) by centrifugation using conditions such as above indicated, washed with 1/10 volume borate buffer, and resuspended in 200 µl water. Thereafter, cells were fixed and their cell walls were permeabilized by mixing such with 800 µl of a methanol-formaldehyde solution to give a final concentration of 72 volume percent methanol and 3.6 volume percent formaldehyde in a resulting aqueous 1 ml cell suspension. After a 10 minute contacting period at room temperature, the resulting cells were separated from the resulting aqueous suspending medium and then pelleted by centrifugation at about 14,000 g for 5 minutes in an Eppendorf microcentrifuge, then washed with 1 ml aqueous borate buffer composition such as above identified and next resuspended in hybridization buffer as above described to give a concentration of approximately 1 x $10^9$ cells/ml. Microscopic examination at 1000x of the resuspended cells showed that cell morphology remained unchanged from that of the starting living cells. Typically, 150 µl of this cell suspension was mixed with 120 µl hybridization buffer and 30 µl borate buffer containing the oligonucleotide probe at a concentration of approximately 7.5 µg/ml unless otherwise specified. Hybridization was generally carried out for 30 minutes at either 37°C or 65°C under continuous shaking. Following hybridization, cells were separated by centrifuging as above described, washed once with 1 ml borate buffer, and then resuspended in 1 ml borate buffer for either fluorescence measurement in a fluorometer or direct visual measurement by visual

observation of individual cells under a microscope at 1000x.

Probe synthesis and labeling: An 18 nucleotide long universal probe was used which had the sequence GWATTACCGCGGCKGCTG (W = A or T; K = G or T), referred to hereinafter as SEQ ID NO: 1, as described in the foregoing Giovannoni et al. article. A 35 nucleotide long E. coli specific probe was used which had the sequence TCAATGAGCAAAGGTATTAACTTTACTCCCTTCCT, referred to hereinafter as SEQ ID NO: 2. A 28 nucleotide long Salmonella specific probe was used which had the sequence specific probe AGCTCACAGCATATGCGCTTTTGTGTAC, referred to hereinafter as SEQ ID NO: 3. Except as indicated, the oligonucleotide probes were synthesized on an Applied Biosystems 380B DNA synthesizer. Mixtures of deoxynucleotides were used for sequence positions where degeneracy was desired.

Fluorophore labels were attached to the synthetic oligonucleotides by a two-stage procedure. In stage one, 5-(3-N-trifluoroacetylaminopropenyl)-5'-dimethoxytrityl-2'-deoxyuridine-3'-(2-cyanoethyl-N,N-diisopropylamino) phosphoramidite which is designated as T* and which has the structure:

(1) 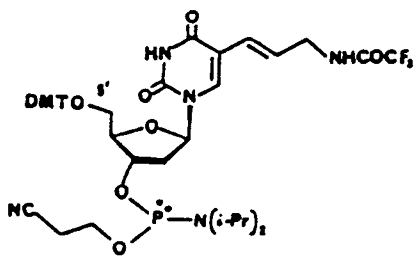

was prepared according to the reference Cook, A.F., Vuocolo, E. and Brakel, C.F. (1988) "Nucl. Acids Res," 16:4077-4095. and then added to the 5'-terminus of the oligonucleotide, while such was still attached to the solid support by the following procedure: A 0.1 M solution of T* phosphoramidite in acetonitrile (100 µl) and a 0.5 M solution of tetrazole in acetonitrile (100 µl) were reacted with the solid support for 5 minutes at room temperature. Conversion of the phosphite to the more stable phosphate linkage was achieved by a 30 second treatment with 50 mM $I_2$ (500 µl) dissolved in a solution of tetrahydrofuran:Lutidine:$H_2O$ (88:10:2). After washing the column with 10 ml $CH_3CN$, the DNA was deprotected by treatment with a 28% solution of $(NH_4)OH$ (55 °C for 16 hr).

In stage two, the crude aminated oligomer (700 µg; appr. 20 $OD_{260}$ units) was reconstituted in 320 µl of 0.1 M $Na_2CO_3$ and 16 µl of triethylamine. After addition of 10 mg (dry powder) of fluorescein isothiocyanate (FITC) (Molecular Probes Inc., Eugene, OR), the mixture was vortexed and incubated at room temperature for 2.5 hr. Most of the unreacted fluorophore was removed by extraction with water saturated butanol. The DNA was purified by reverse-phase HPLC (Vydac protein and peptide C18 column) with a 0-30% acetonitrile gradient in 100 mM triethylammonium acetate buffer, pH 7.0.

For labeling with Texas Red (T-Red), 25 O.D. of the crude oligomer was reconstituted in 500 µl of 0.05 M sodium borate, pH 9.3 and mixed with 10 mg of Texas Red (T-353; Molecular Probes Inc., Eugene, OR). After an 8 hour incubation at room temperature, the mixture was extracted with water saturated butanol to remove unreacted T-Red and was evaporated to dryness. The DNA was then purified by electrophoresis through a 20% polyacrylamide gel (1% crosslinker), eluted into buffer (100 mM TRIS-HCl, pH 8.0, 500 mM NaCl, 5 mM EDTA), and desalted by passage through a Waters Sep-pac cartridge.

Cell detection and quantitation by fluorometry: Fluorescence determinations were done on a SPF-500 SLM AMINCO Spectrofluorometer in a 1 cm path cuvette with an input voltage of 1200V. Excitation was at 494 nm for FITC and 596 nm for T-Red. Emission was determined at 516 nm and 602 nm respectively. For quantitation the relative emission of labeled probe molecules were determined at a concentration of approximately 0.7 µg/ml in borate buffer. The number of probes per cell was calculated according to the formula below without correcting for quench.

$$(2) \qquad P = \frac{E_t - E_a}{E_p \times N},$$

wherein:

P = number of probe molecules per cell,

$E_t$ = total emission per sample,
$E_a$ = autofluorescence of sample,
$E_p$ = emission per probe molecule, and
N = number of cells per sample.

Cell detection by flow cytometry: Cell sorting as carried out on an EPICS 752 Flow Cytometer (Coulter Electronics) at the University of Illinois Biotechnology Center (Champaign-Urbana).

Cell detection by fluorescent microscopy: Smears of stained cells were observed under oil with a Zeiss Axioskop. Fluorescence was observed with a blue filter (Zeiss H485) for FITC, and a green filter (Zeiss H546) for T-Red.

Factors affecting fluorescent intensity of cells stained by in situ hybridization in suspension: Conceptually, the fluorescent intensity of cells stained by in situ hybridization in aqueous suspension should be dependent on a variety of factors, such as the choice of probe and fluorophore, the permeability of the cells, the hybridization conditions, and the number of available target sequences in a cell. Since the probes used were targeted toward 16S ribosomal RNA, which may be present in up to 100,000 copies per actively growing bacterial cell per DeLong, E.F., Wickham, G.S. and Pace, N.R. (1989) "Phylogenetic stains: Ribosomal RNA-based Probes for the Identification of Single Cells", Science 243: 1360-1363, the number of these molecules accessible for hybridization was evaluated. The results are shown in Fig. 6.

Fig. 6 shows that when the hybridization reaction was driven by a sufficiently high probe concentration, the number of probes per cell can approach the theoretical maximum. This data suggests that virtually all 16S rRNA molecules are available for hybridization under the present process conditions. Moreover, this data suggests that the nucleotide sequence to which the 18 nucleotide-long universal probe hybridizes is not involved in intra-strand secondary structure formation, which is consistent with the secondary structure model proposed by Stern S., Weiser, B. and Noller, H.F. (1988) "Model for the three-dimensional folding of 16S ribosomal RNA." J. Mol. Biol. 204: 447-481.

Moreover, Fig. 6 shows that the actual number of target molecules that undergo hybridization with this probe is strongly dependent on probe concentration (Fig. 6). The fraction of total probes that was sequestered by the cells during the hybridization averaged between about 20% and 30% of input probes as long as the total number of ribosomes exceeded the total number of probe molecules in the reaction mixture.

The effect of hybridization time was evaluated and the results are shown in Fig. 7. The data indicates that, for the small universal probe, the hybridization reaction is fairly rapid. A significant amount of hybridization occurred in the first few seconds, and the reaction was essentially complete after about 30 minutes. Contacting times in excess of about 1 hour resulted in reduced hybrid formation for unknown reasons.

The effect of hybridization time and probe concentration on the hybridization reaction was evaluated and the results are shown in Fig. 8 and Fig. 9 for two different probes, the 18 nucleotide-long universal probe, and the 35 nucleotide-long E. coli specific probe using hybridization times of 30,60 and 90 minutes at 37°C. For the universal probe, there was no difference between each of 30, 60 and 90 minute hybridization times, and the reaction appeared to depend completely on probe concentration (Fig. 8). For the 35 nucleotide-long E. coli probe, however, both the reaction time and the probe concentration were found to have a significant effect on the hybridization, suggesting that the larger probe was hindered in its diffusion into the cell (Fig. 9). This conclusion is further supported by comparing the universal and E. coli specific probe by hybridizing each such probe to an equal number of either E. coli or Proteus cells.

Fig. 11 shows that the universal probe hybridized to both cell types equally well in speed and amount, while the E. coli specific probe required more time, but hybridized only to E. coli cells. Further evidence for such probe specificity is shown below.

Methods of detecting cells by in situ hybridization in aqueous suspension and their sensitivity: Since the probes used herein were tagged with fluorophores, cells stained by in situ hybridization can be detected by any method that allows measurement of fluorescence. In the evaluations described above, cells were hybridized and their fluorescence determined in aqueous dispersion. The minimum number of E. coli cells required for detection was on the order of about $10^7$ per ml when the universal probe with a single fluorophore per probe molecule was used, independent of the type of fluorophore involved (Fig. 10). However, since more than one fluorophore can be added per probe molecule, and since more than one type of probe can be used simultaneously, the sensitivity of this detection method can be readily increased several orders of magnitude.

The specificity of the present in situ hybridization reaction, that is, the ability of a specific oligonucleotide probe to discriminate between target and non-target cells, was evaluated and the results are shown in Fig. 11. The work reported and illustrated in the plots of Fig. 11 utilized (a) Proteus cells

hybridized by the universal probe, (b) Proteus cells hybridized by the E. coli. specific probe, (c) E. coli. cells hybridized by the universal probe, and (d) E. coli. hybridized by the E. coli. specific probe. The curves obtained for each of (a) and (c) fall on the same solid line shown in Fig. 11. As expected, the universal probe binds equally well to Proteus and E. coli cells, while the E. coli specific probe hybridizes only to E. coli cells, but not to Proteus cells.

For application of the present diagnostic method to food or clinical usage, the capability of this method for detecting certain types of cells in admixture with non-target cells under aqueous dispersion conditions was conducted. Table II (below) shows the fluorescence obtained after hybridizing a mixture of E. coli and Proteus cells with an E. coli specific probe. As few as 3-4 x $10^6$ E. coli cells were detected in the presence of a 20 fold excess of Proteus cells with the fluorometer. The present inventive method is therefore well suited for use as a diagnostic procedure.

TABLE II

| EFFECT OF NON-TARGET CELLS ON DETECTION SENSITIVITY | |
|---|---|
| E. coli cells (%) | Relative Fluorescence of cell mixture in instrument units) |
| 100.0 | 6.52 |
| 59.2 | 7.11 |
| 22.5 | 1.9 |
| 5.5 | 0.83 |
| 1.2 | 0.53 |
| 0.23 | 0.31 |
| 0.0 | 0.32 |

In the procedure used for obtaining the results shown in Table II, a decreasing number of E. coli cells was mixed with 6.7 x $10^7$ Proteus cells and probed with the E. coli specific probe using the procedural steps of this invention.

A more sensitive detection procedure involved the counting of single cells using the fluorescent activated cell sorter above identified herein. When E. coli and Proteus cells were mixed after hybridization to the 35 nucleotide long E. coli specific probe, as few as 5 x $10^5$ E. coli cells/ml in the presence of a 100 fold excess of non-target cells were detected by the cell sorter with a signal to noise ratio of 3.

A further sensitive detection method utilizes direct visual observation of stained cells by fluorescent microscopy. Mixtures of E. coli and Proteus cells were hybridized simultaneously using two probes, the universal probe tagged with T-Red, and the E. coli specific probe tagged with FITC.

Aliquots (5 $\mu$l) of the hybridization mixtures were dried onto slides, in accord generally with prior art microscopy protocols, and viewed under the microscope with the appropriate filter sets to allow visualization of either the red or green fluorophore. Since all cells were stained red by the universal probe, but only E. coli cells were stained green by the E. coli specific probe, E. coli cells were detected specifically when in mixture with an up to 10,000 fold excess of Proteus cells. The minimum concentration of E. coli cells necessary for microscopic detection of such cells without a time consuming scan of the microscope slide was found to be approximately $10^4$ cells/ml. Thus, the present method is well suited for use as a diagnostic procedure with mixed bacterial species and a plurality of different probe types using microscopy and fluorophore labeled probes.

Characterization of cells containing hybridized nucleic acids: The foregoing illustrative detection procedures reveal the presence of treated cells containing cellular nucleic acids which have been hybridized with the oligonucleotide probes. In all cases, the individual bacterial cells were first fixed and their cell walls permeabilized by preliminary treatment with 72 volume percent methanol and 3.6 volume percent formaldehyde after which in situ hybridization with labeled oligonucleotide probes was carried out as described herein under aqueous liquid phase conditions involving cellular targeted nucleic acids.

The metabolic state of the cell affects in situ hybridization: The intensity with which a stained hybridized cell produced by the methods herein provided fluoresces was found to be dependent on the number of probe molecules that become sequestered within the cell by in situ hybridization to ribosomal RNA molecules. Since the number of ribosomes per cell is regulated by its metabolic state, that is, the growth rate of the cell, it is expected that the fluorescent intensity would vary also with the condition of the cell.

This situation was evaluated and the results are shown in Fig. 12. Monitoring cell density by measuring the optical density at 550 nm ($OD_{550}$) revealed a typical growth curve with an initial lag phase preceding

logarithmic growth that was followed by cessation of growth and the early stationary phase. The number of ribosomes per cell was expected to be low during both the lag phase and early stationary phase, but high during the log phase. When aliquots of the culture were stained by in situ hybridization, a spike of fluorescence at the 135 minute time point suggested that the cells were the brightest when stained during the log phase, which was shown to be the case by photographs. These results confirm the expectation above indicated.

Application of in situ hybridization to the diagnosis of infectious disease: The rapidity and specificity of the present technique for in situ hybridization, combined with its capability of single cell resolution by fluorescence microscopy, or capillary electrophoresis provides a useful detection method for diagnosis of bacterial infections. Such a diagnostic technique is especially suitable in use as a replacement procedure for the standard prior art medical microbiological identification procedure that is directed toward isolation of a specific bacterial strain as the causative organism, such as E. coli in female urinary tract infections, or Salmonella intestinal infections. Diagnosis by in situ hybridization in aqueous suspension as taught herein avoids the need for bacterial strain isolation and identification by culture as taught in the prior art.

A first study of the in situ diagnostic procedure of this invention was carried out on urine specimens that were obtained from the clinical microbiology laboratory of Bernard Mitchell Hospital (University of Chicago). The samples were each between 1 and 2 days old and were first separately analyzed by the clinical laboratory. Because of the age of these specimens at the time of the present evaluation, each sample was diluted in broth and cultured for 2 to 3 hours. In situ hybridization was then performed on each sample in accord with the presently preferred procedure of this invention as above described. Probe/treated cell contacting was carried out simultaneously with the 18 nucleotide long universal probe, labeled with T-Red, and the 35 nucleotide long E. coli specific probe, labeled with FITC, as described above.

The procedural steps and time required for each step are shown in Table III below. These steps and times are also inclusive of the steps and times in other experimental work described herein unless otherwise stated.

TABLE III

TIME REQUIRED FOR DIAGNOSIS PROCEDURE

| Step No. | Step | Laboratory Prototype Time Duration (Range in minutes) | Expected Optimized Clinica Laboratory Time Duration (Range in minutes |
|---|---|---|---|
| 1. | Specimen dilution activation (if used) and/or culture | 2 - 120 | 40 - 90 |
| 2. | Centrifugal separation and cell resuspending in borate buffer wash | 8 - 10 | 8 - 10 |
| 3. | Recentrifuging and cell suspending in $CH_3OH/CH_2O$ treating composition | 12 -15 | 12 - 15 |
| 4. | Recentrifuging, washing and cell suspending in TRIS-HCl hybridization buffer and probe contacting | 38 - 40 | 30 - 40 |
| 5. | Recentrifuging and cell suspending in borate buffer wash | 6 - 8 | 6 - 8 |
| 6a. | Recentrifuging and spreading cells on microscope slide | 6 - 8 | 1 - 2 |
| 7a. | Detecting fluorophores in hybridized cells under fluorescence microscope | 1 - 5 | 1 - 3 |
| (In place of steps 6a and 7a the following alternate steps are used:) | | | |
| 6b. | Recentrifuging and resuspending cells in borate buffer | 6 - 8 | 6 - 8 |
| 7b. | Detecting fluorophores in hybridized cell with fluorometer | 1 - 2 | 1 - 2 |
| Total time (with steps 6a and 7a) | | 73 - 206 | 98 - 168 |
| Total time (with steps 6b and 7b) | | 73 - 203 | 103 - 173 |

The results obtained in the diagnosis of each specimen by the procedure of this invention were approximately equivalent to those obtained by the diagnosis previously made by the clinical laboratory. In a specific but typical sample, E. coli was identified among other cells (>$10^5$ E. coli,>$10^5$ Klebsiella or Enterobacter, and <$10^4$ gram positive organisms). When viewed under a blue filter, the green appearing E. coli cells were easily identified. When shifting to a green filter, all cells were seen, since they were all stained with the universal probe. Since the culture of these urine samples was for 2 hr only, which was sufficient for cell activation but which did not result in a significant increase in cell number, in situ hybridization in accord with the present invention not only achieved a rapid identification of a specific organism (total laboratory prototype diagnostic procedure processing time was approximately 3 hours, including incubation), but also concurrently achieved an estimate of cell concentration in the urine

specimen. Diagnosis time is thereby reduced from a typical 2 day prior art urine diagnostic procedure, such as now used clinically in the prior art, to a new diagnostic procedure requiring when optimized not more than about 2 to 3 hours.

A second study of the in situ diagnostic procedure of this invention was performed by spiking fecal samples with a clinical isolate of Salmonella. The spiked samples were diluted into a GN broth tube and incubated for 3.5 hour at 37°C. In situ hybridization in aqueous suspension was then performed on each sample in accord with the presently preferred procedure of this invention as above described. The hybridization temperature was 65°C. All hybridizations were carried out with a combination of the universal probe (FITC labeled) and the Salmonella specific probe (T-Red labeled) as described above. The relatively few Salmonella cells present were easily identified among the overwhelming number and variety of gram negative microorganism flora present, such flora being characteristic of the human gut. No subculture was necessary for diagnosis. The results were obtained using a laboratory prototype diagnostic procedure in about 3-4.5 hours (including incubation) which is in marked contrast to the 3 day fecal diagnostic procedure such as now used clinically in the prior art.

Painting by in situ hybridization in aqueous suspension: For many organisms, specific genes have been identified and described that are characteristic for that organism or a class of organisms. For an example, the presence of the gene for beta-galactosidase is characteristic for organisms that can metabolize (utilize) lactose by degrading it into glucose and galactose with aid of the enzyme beta-galactosidase. This gene is characteristic for some members of the family of Enterobacteriaceae. This gene, or its transcript (mRNA), can serve as the starting target material for a set of probes that allow "painting" of this gene, or its transcripts, by in situ hybridization.

A presently preferred method for probe production involves cloning the gene, or a c-DNA copy of the transcript (mRNA) into a vector, producing large quantities of the vector DNA by fermentation, or large quantities of the mRNA by transcription. This product DNA or RNA is then conveniently broken into small pieces of about 20 to about 100 nucleotides in length by mechanical means, such as for example, by sonication, or by partial enzymatic digestion with the appropriate nucleases. The DNA or RNA pieces are then labeled with one or more of the known appropriate label moieties by chemical or enzymatic methods such as are well known to the art. These labeled DNA or RNA fragments are then used as "painting probes" in the in situ hybridization procedure of this invention. This has already been used in the prior art to produce "painting probes" for whole human chromosomes, which were then used in in situ hybridization to slide-fixed metaphase nuclei. When the DNA sequence of the gene is known, the probes can be produced, if desired, synthetically on a DNA synthesizer.

This was accomplished in the following experiment, which serves as an example of the "painting" of target sequences, such as low copy number cellular nucleic acid components, with multiple probes using in situ hybridization of cells in aqueous suspension.

The cellular nucleic acid target was 16S rRNA of the bacterium E. coli. Three oligonucleotide sequences were each made on a DNA synthesizer, each containing a sequence complementary to a different region on the 16S rRNA molecule. Each such sequence was labeled with one fluorophor (FITC). The probes were used both individually and in combination.

Such bacterial cell species (E. coli) was contacted with a treating composition that comprises on a 100 volume percent basis about 72 volume percent methanol, about 3.6 volume percent formaldehyde, and about 24.4 volume percent water, as above described, then centrifugally separated therefrom, and resuspended in an aqueous hybridization buffer, as herein above described in the presently preferred procedure.

The three probe types are used separately and also as a mixture comprised of equal amounts of each probe. The contacting procedure was the same with the treated bacterial species. During such contacting, which is carried out with gentle continuous agitation, the concentration of bacteria was about $3 \times 10^9$ cells per ml, the total probe concentration was about $6 \times 10^{14}$ molecules per ml, the contacting temperature was about 37°C and the contacting time was about 30 minutes. Thereafter, the cells are separated by centrifugation, washed and resuspended in fresh aqueous borate buffer as herein above described.

The resuspended cells are then passed through a fluorometer and fluorometer readings are taken. Results are shown in Table IV below:

TABLE IV

| FLUORESCENCE OF PAINTING PROBES | | |
|---|---|---|
| Probe | Fluorometer Readings | Fluorometer Readings Minus Background |
| no probe | 0.149 | -- |
| A | 1.485 | 1.336 |
| B | 1.00 | 0.851 |
| C | 1.44 | 1.291 |
| A+B+C | 3.675 | 3.526 |

Table IV shows that the fluorescence obtained by painting is additive under the conditions employed. Using additional probes targeted at different regions of the 16S rRNA molecules would further increase the fluorescence of the resulting hybridized sample. Painting appears to be particularly well suited for diagnosis of low copy number cellular nucleic acid sequences.

Amplification by in situ hybridization in aqueous suspension: Amplification of a fluorophore-labeled hybrid involving a low copy number cellular nucleic acid is achievable by various procedures. For example, there is first synthesized a plurality of probes on a DNA synthesizer such that the probes can hybridize to each other serially. Each such probe can have a single label moiety, preferably a fluorophore. Thus, a primary probe is prepared to contain two regions (A) and (B). Region (A) is complementary to a sequence on the nucleic acid target, and region (B) provides a different sequence for hybridizing such primary probe to a secondary probe. The secondary probe, in turn, is prepared to have two regions (C) and (D). Region C is complementary to region B of the primary probe, and region D provides a sequence for hybridizing to a tertiary probe. The tertiary probe has two regions (E) and (F), region (E) being complementary to region (D), and region (F) providing a sequence for hybridizing to a fourth probe. The fourth probe has two regions (G) and (H). Region (G) is complementary to its region (F) and region (H) provides a sequence for hybridizing to a fifth probe. In this example, the fifth probe contains a single sequence (I) which is complementary to region (H). Theoretically, further similar probes each containing two such chain-forming oligonucleotide sequences could be used also.

To evaluate such a procedure, five such sequenced probes were first formed and identified successively as types: P, S, T, R, and U. The sequence region (A) of the primary probe (P) was complementary to a sequence on the 16S rRNA molecule of E. coli.

Such bacterial cell species (E. coli) is preliminarily contacted with a treating composition comprised on a 100 volume percent basis of about 72 volume percent methanol, about 3.6 volume percent formaldehyde, and about 24.4 volume percent water as above described, then centrifugally separated therefrom, and resuspended in an aqueous hybridization buffer as hereinabove described.

All of such probe types were used separately or were first mixed in the combinations shown below in the hybridization buffer, incubated at 47°C for 45 minutes, and then were contacted with such so treated bacterial species (E. coli) under aqueous liquid phase conditions with gentle continuous agitation. During each contacting, the concentration of such bacteria was about $3 \times 10^9$ cells per ml, the concentration of each probe was about $6 \times 10^4$ molecules per ml, the contacting temperature was about 37°C, and the contacting time was about 30 minutes.

At the end of each such contacting, the residual probes of each indicated type are separated from the resulting cells by centrifuging, followed by washing and resuspending in fresh such aqueous borate buffer, such as herein above described.

Each such so resuspended batch of so contacted cells is passed through a fluorometer and fluorometer readings are taken. The results are shown in Table V below:

TABLE V

| EFFECT OF AMPLIFICATION PROBES | | | |
|---|---|---|---|
| Probe | Total Probe Length In Nucleotides | Fluorometer Readings | Fluorometer Readings Minus Background* |
| no probe | -- | 0.134 | -- |
| P | 56 | 0.796 | 0.662 |
| S | 40 | 0.352 | 0.218 |
| T | 40 | 0.322 | 0.188 |
| R | 40 | 0.566 | 0.432 |
| U | 20 | 0.467 | 0.333 |
| P+S | 76 | 1.46 | 1.326 |
| P+S+T | 96 | 1.55 | 1.416 |
| P+S+T+R | 116 | 1.87 | 1.736 |
| P+S+T+R+U | 116 | 2.1 | 1.966 |

Table V footnote: *In the readings shown, the "no probe" value shown under "Fluorometer Readings" was subtracted.

The data presented in Table V shows that amplification of cellular nucleic acid sequences is achieved. When used individually, such primary probe P gives a net fluorescence of 0.662 instrument units (arbitrary), while the secondary probe S and the subsequent successive probes identified as T, R, and U give significantly less net fluorescence. The secondary and subsequent probes should not give any net fluorescence, but apparently such probes bound in this evaluation non-specifically to some nucleic acid sequences in the cells. However, when probes P and S were mixed and pre-annealed, the fluorescence doubled as expected. Additions of subsequent probes to the P-S complex do not increase the fluorescence, but instead lead to a relative decrease. This suggests that the probe complex has become too large to enter the cells, or perhaps is too large to enter the ribosome and hybridize to the target sequence. Probes sizes of less than about 100 as nucleotides are preferred.

However, sequence modifications of the probes can be made to reduce the background binding. Also, probes can be used sequentially (i.e., not pre-annealed with intermittent centrifugal separations and washings to remove the proceeding probe) to avoid complex formation before the probes enter the cells. Finally, cellular nucleic acid targets other than ribosomal RNA molecules probably would be less size constricted and thus allow for hybridization of larger probe complexes within a cell. Amplification appears to be particularly well suited for diagnosis of low copy number cellular nucleic acid sequences.

Use of radioactively labeled probe: The 18 nucleotide long universal probe (above identified) was radioactively labeled by enzymatic transfer of the gamma-$^{32}$P group from gamma-$^{32}$P-ATP to the 5'-end of the oligonucleotide by well established protocols. The labeling reaction was such that 4.3 $^{32}$P molecules were attached per $10^4$ probe molecules; i.e., only approximately 4 in 10,000 probe molecules was made radioactive. Approximately $7 \times 10^{14}$ such labeled probe molecules were contacted with approximately $10^8$ E. coli cells that had been prepared for in situ hybridization in aqueous suspension by the herein described method for 30 minutes at 37°C in a total volume of 300 ul. After this time, 1 ml of $H_2O$ was added to this reaction mixture and the cells were then separated from the free probe molecules by centrifugation. The resulting cell pellet was resuspended in 100 ul $H_2O$, and 50 ul of that was mixed with scintillation fluid (5 ml) and counted in a commercially available scintillation counter (Beckmann Instruments). The sample described here gave 11634 CPM (counts per minute); i.e., 0.000232 CPM per cell. Since 500 CPM can be reliably detected with this technique, the assay sensitivity in this example was approximately $2^{x}10^6$ E. coli cells. However, by using fresh radioisotope (the one used here was over 2 weeks old, which means 50% disintegrated) the sensitivity could be improved 2 fold, and by preparing a probe with higher specific activity (i.e., one $^{32}$P molecule per probe), the sensitivity could be improved 2500 fold. This would allow detection of less than 500 bacterial cells. Thus, by in situ hybridization in aqueous suspension with radioactively labeled probes, a small number of bacterial cells can be detected very rapidly (this experiment took appr. 1 hour and should take 2 to 3 hours if cells had to be activated), which makes this method very suitable for rapid bacterial diagnostics. Furthermore, this work demonstrates the versatility of the in situ hybridization in aqueous suspension technique.

Effect of methanol-formaldehyde concentration on probe penetration: E. coli cells were grown to an optical density of $O.D._{550}$ = 0.2 (appr. $10^8$ cells/ml). A 10 ml aliquot of the cell culture was centrifuged and

the pelleted cells were resuspended in 10 ml of 50 mM TRIS-HCl, pH 7.0, and 500 mM NaCl. Samples of such resuspended cells (300 ul each) were again centrifuged, and the cell pellets were resuspended in a mixture of hybridization buffer and methanol-formaldehyde solution as shown below in Table IV. The methanol-formaldehyde solution consisted of 9 parts 100% (volume) methanol and 1 part of aqueous 44.4% (volume) formaldehyde. After 10 minute incubation at room temperature, the cells were pelleted by centrifugation and resuspended in 30 ul hybridization buffer containing 1 ug of an E. coli specific oligonucleotide probe labeled with FITC as above described. After incubation for 30 minutes 37°C, the resulting cells were pelleted by centrifugation, washed with 50 ul buffer (187.5 mM NaCl, 25 mM TRIS-HCl, pH 7.8, 1.25 mM EDTA), resuspended in 300 ul of the same buffer, and incubated at 37°C for 40 minutes. After that time, the resulting cells were pelleted by centrifugation and resuspended in 30 ul 50 mM sodium tetraborate buffer (pH not adjusted). Samples of that composition were further diluted in tetraborate buffer for fluorescence measurements in a SLM Model 4800 Spectrofluorometer modified for photon counting (SLM-Aminco). Other such samples were used for absorbance measurements at 550 nm in order to determine cell density.

TABLE VI

| EFFECT OF METHANOL-FORMALDEHYDE PRE-TREATMENT ON CELLS UNDERGOING IN SITU HYBRIDIZATION IN AQUEOUS SUSPENSION | | |
|---|---|---|
| Sample # | Hybridization Buffer | Methanol-Formaldehyde Solution |
| 1 | 1000 | 0 |
| 2 | 800 | 200 |
| 3 | 600 | 400 |
| 4 | 400 | 600 |
| 5 | 200 | 800 |
| 6 | 0 | 1000 |

This data was used in the preparation of Figs. 13 and 14.

Other and further embodiments of this invention will be apparent to those skilled in the art from the foregoing teachings from which no unnecessary or undesirable limitations are to be implied or inferred.

## Claims

1. A method for determining the presence of a microorganism selected from the group consisting of prokaryotes. and yeasts by in situ hybridization in aqueous suspension comprising the steps of:
(a) first contacting said microorganism with an aqueous treating composition which fixes said microorganism and also permeabilizes the cell wall thereof to an extent sufficient to permit penetration therethrough of an oligonucleotide without appreciably altering the cellular morphology of said microorganism;
(b) secondly contacting said so first contacted microorganism while in an aqueous dispersion with at least one labeled nucleic acid probe, each said probe being permeable through said permeabilized cell wall and incorporating a nucleotide sequence that is complementary to a particular nucleotide sequence that exists in a cellular nucleic acid component in said microorganism, thereby to cause hybridization of said cellular nucleic acid component with said probe; and thereafter
(c) detecting in said so secondly contacted microorganism said hybridized nucleic acid component(s) if detectably present using said probe label.

2. The method of claim 1 wherein one or two said labeled nucleic acid probes are used whose nucleic acid portion comprises oligonucleotides having nucleotide sequences that are complementary to particular nucleotide sequences of one or more cellular nucleic acid components of said microorganism, wherein said probe contains one or two label moieties per probe molecule.

3. The method of claim 1 wherein between said first contacting and said second contacting steps, the resulting so fixed and so permeabilized microorganism is separated from the associated aqueous medium by centrifuging and then is resuspended in another aqueous medium, thereby separating said treating composition from said resulting microorganism, wherein, after said first contacting and before the end of said second contacting, said microorganism is heated to a temperature in the range of about 80 to about 100°C for a time in the range of about 1 to about 5 minutes, and wherein between said second contacting and said detecting steps, the resulting so probe contacted microorganism is

separated from the associated aqueous medium by centrifuging, and said so separated microorganism is resuspended in another aqueous medium.

4. The method of claim 1 wherein said nucleic acid probes are labeled with moieties which must be further treated with identifying agents before said detecting is completed, comprising the group including enzymes, wherein said detecting is carried out by first admixing with the resulting said so secondly contacted microorganism under aqueous liquid phase conditions a chemiluminescent precursor substrate which is converted by said enzymes into a chemiluminescent agent which emits photons, and then detecting such photon emission by at least one procedure that is selected from the group consisting of microscopic observation, image analysis, and luminometry.

5. The method of claim 4 wherein said probe label moieties comprise at least one label selected from the group consisting of biotin and hapten, wherein said detecting is carried out by first admixing with said resulting so secondly contacted microorganism under aqueous liquid phase conditions at least one agent selected from the group consisting of avidin, streptavidin and antibodies said agent being itself labeled with at least one label moiety selected from the group consisting of fluorophores, radioisotopes and chromogens, thereby to react said agent with said probe labels, then removing residual extracellular amounts of said agent, and thereafter detecting the presence of said agent label moiety.

6. The method of claim 4 wherein said probe label moieties comprise at least one label selected from the group consisting of avidin, streptavidin, and antibodies, wherein said detecting is carried out by first admixing with said resulting so secondly contacted microorganism under aqueous liquid phase conditions at least one agent selected from the group consisting of biotin and hapten, said agent being itself labeled with at least one label moiety selected from the group consisting of fluorophores, radioisotopes, and chromogens, thereby to react said agent with said probe labels, then removing residual extracellular amounts of said agent, and thereafter detecting the presence of said agent label moiety.

7. The method of claim 1 wherein, in said second contacting, at least two different labeled nucleic acid probes are used, and one of said probes incorporates a nucleotide sequence which is common to one or two species of bacteria, and a second of said probes incorporates a nucleotide sequence which is specific to one of the same or another species of bacteria, and wherein each of said probes incorporates a different label portion, whereby cellular hybrids formed with said different probes in admixture can be separately detected during said detecting.

8. The method of claim 1 wherein, in said second contacting, at least two different labeled nucleic acid probes are used, and one of said probes incorporates a nucleotide sequence which is specifically complementary to a nucleotide sequence of the ribosomal RNA in one species of bacteria, and a second of said probes incorporates a nucleotide sequence which is specifically complementary to a nucleotide sequence of a DNA found in the same one species of said bacteria, said DNA being selected from the group consisting of plasmid DNA and chromosomal DNA.

9. The method of claim 1 wherein, in said second contacting, at least two different labeled nucleic acid probes are used, and at least two of said probes are targeted for the same nucleic acid type that exists in a given microorganism, said nucleic acid being selected from the group consisting of messenger RNA, plasmid DNA and chromosomal DNA, each of said probes containing a different nucleotide sequence which is complementary to a particular nucleotide sequence along the molecule of said target nucleic acid type, whereby, during said second contacting, hybridization of said probes to the same said target nucleic acid type can occur, thereby enhancing detectability of said target nucleic acid during said detecting.

10. The method of claim 1 wherein, in said second contacting, more than two different labeled nucleic acid probes are used at least two of which each incorporate two different oligonucleotide sequences, a primary one of said probes having one nucleotide sequence which is complementary to a cellular target nucleic acid nucleotide sequence and also having a second nucleotide sequence which is complementary to one nucleotide sequence of a secondary one of said probes, said secondary one of said probes having said one nucleotide sequence which is complementary to said second nucleotide sequence of said primary probe and also having a second nucleotide sequence which is complementary to one

nucleotide sequence of a tertiary labeled probe, whereby, during said second contacting, hybridization of said primary probe to said target nucleic acid nucleotide sequence can occur, hybridization of said secondary probe to said primary probe can occur, and hybridization of said tertiary probe to said secondary probe can occur, thereby enhancing detectability of said target nucleic acid during said detecting.

**11.** A method for identifying the presence of a particular species of microorganism selected from the class consisting of bacteria and yeasts in a microorganism population that may include members of other microorganism species, said method comprising the steps of:

(a) contacting a representative group of said microorganism population with a treating composition which comprises about 72 weight percent methanol and about 3 weight percent formaldehyde, thereby to fix and cell wall permeabilize said particular species of microorganism that is to be identified if such species is present;

(b) centrifugally separating said contacted group of microorganisms from said treating composition;

(c) redispersing said so separated group of microorganisms in an aqueous medium;

(d) admixing with said so redispersed group of microorganisms at least one labeled oligonucleotide probe which incorporates in its oligonucleotide portion a nucleotide sequence that is complementary to a particular nucleotide sequence which is present in a nucleic acid component of said particular species of microorganism that is to be identified, whereby said oligonucleotide probe traverses said permeabilized cell walls and hybridizes with said particular nucleotide sequence if said particular species of microorganism is present in said redispersed group of microorganisms;

and

(e) detecting the presence in the resulting so probe contacted and redispersed group of microorganisms hybridized nucleic acid components if present, wherein, between said steps (d) and (e), said so probe-admixed group of microorganisms is centrifugally separated from said aqueous medium and from residual quantities of said oligonucleotide probe therein, and said so separated group of microorganisms is redispersed in another aqueous medium and said nucleic acid component is selected from the group consisting of ribosomal RNA, messenger RNA, transfer RNA, chromosomal DNA, and extra-chromosomal DNA.

**12.** The method of claims 1, 2 and 11 wherein said nucleic acid or oligonucleotide portions comprise an oligomeric DNA containing about 8 to about 40 nucleotides having attached a label portion comprised of at least one moiety selected from the class consisting of fluorophore-containing groups, chromogen-containing groups and radioisotope-containing groups.

**13.** The method of claims 1 and 11 wherein said probe incorporates at least one fluorophore-containing group per probe molecule as the label portion of said probe, wherein said detecting is accomplished by microscopic examination of said so probe admixed cells in the presence of light having a frequency which causes said fluorophore containing groups in hybridized microorganisms to fluoresce when present, wherein said detecting is accomplished by fluorimetry, capillary electrophoresis or with a fluorometer, and wherein said nucleic acid component is selected from the group consisting of messenger RNA, transfer RNA, chromosomal DNA, and extra-chromosomal DNA.

**14.** The method of claim 1 and 11 wherein the label portions of said nucleic acid probes comprise radioisotopes, and wherein said detecting is carried out by at least one procedure selected from the group consisting of (a) filtration and radioactivity quantitation, and (b) scintillation counting.

**15.** The method of claim 14 wherein said microorganism population is derived from urine, wherein said microorganism population in said urine is so admixed with such a labeled probe whose oligonucleotide portion is complementary and species specific to a cellular nucleic acid component in E. coli.

**16.** The method of claim 14 wherein said microorganism population is derived from fecal matter, wherein said microorganism population in said fecal matter is so admixed with such a labeled probe whose oligonucleotide portion is complementary and species specific to a cellular nucleic acid component in Salmonella.

**17.** A microorganism selected from the class consisting of gram negative bacteria and yeasts,
which has been fixed,

whose cell wall has been permeabilized to an extent sufficient to permit penetration therethrough of an oligonucleotide,

whose cellular morphology after said fixing and said permeabilizing is not appreciably altered, and

which is centrifugable without alteration in cellular morphology and without substantial cell loss, and

wherein at least one nucleic acid component thereof contains at least one particular nucleotide sequence taken from the group including, ribosomal RNA, messenger RNA, transfer RNA, and plasmid DNA, chromosomal DNA, cellular extra-chromosomal DNA that is derived from a member of the group consisting of phage DNA and viral DNA that has been hybridized with an oligonucleotide probe that includes a nucleotide sequence that is complementary to said particular nucleotide sequence.

FIG. 1

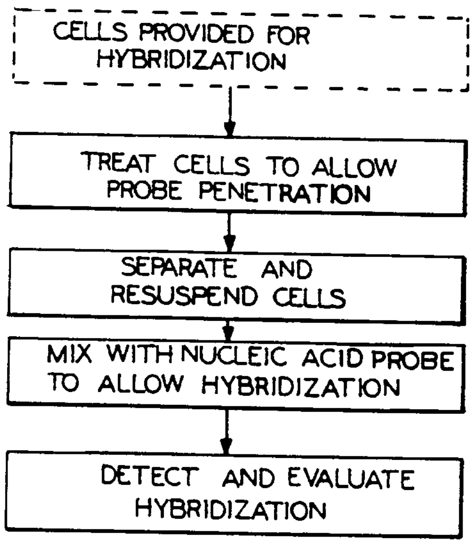
CELLS PROVIDED FOR HYBRIDIZATION
TREAT CELLS TO ALLOW PROBE PENETRATION
SEPARATE AND RESUSPEND CELLS
MIX WITH NUCLEIC ACID PROBE TO ALLOW HYBRIDIZATION
DETECT AND EVALUATE HYBRIDIZATION

FIG. 2

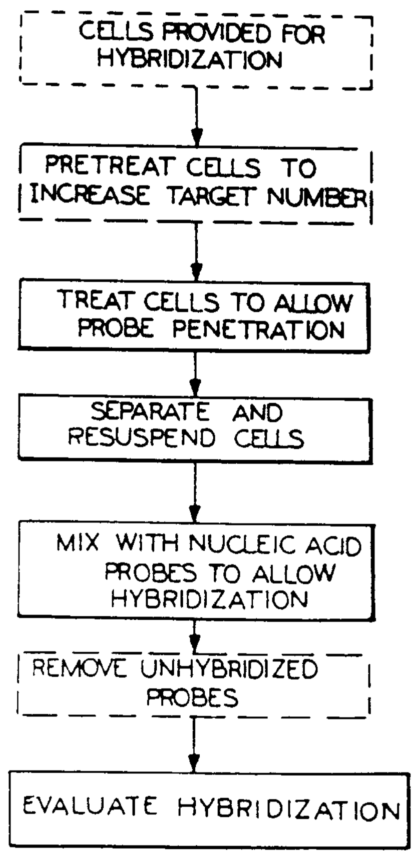
CELLS PROVIDED FOR HYBRIDIZATION
PRETREAT CELLS TO INCREASE TARGET NUMBER
TREAT CELLS TO ALLOW PROBE PENETRATION
SEPARATE AND RESUSPEND CELLS
MIX WITH NUCLEIC ACID PROBES TO ALLOW HYBRIDIZATION
REMOVE UNHYBRIDIZED PROBES
EVALUATE HYBRIDIZATION

FIG. 3

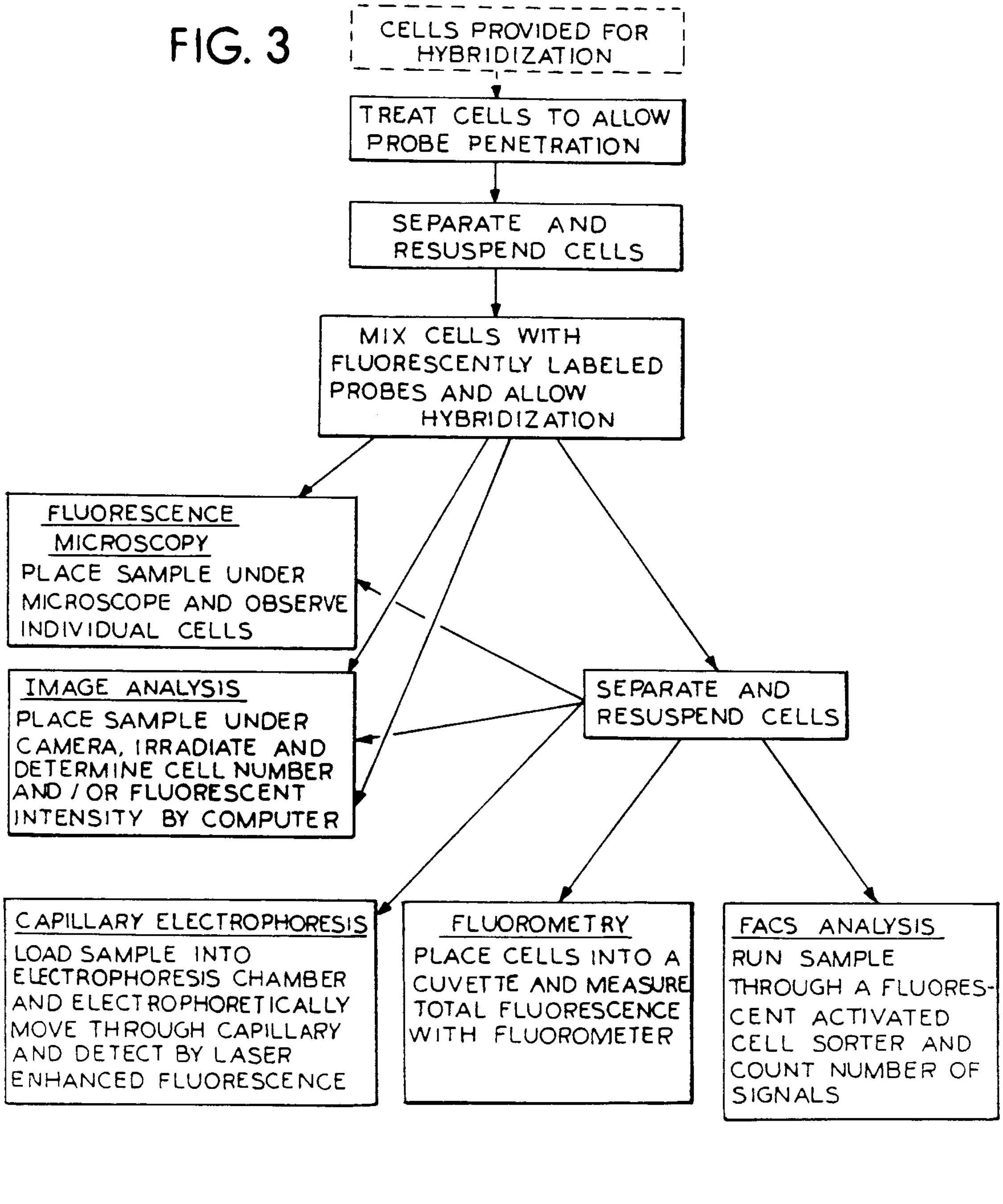
CELLS PROVIDED FOR HYBRIDIZATION
TREAT CELLS TO ALLOW PROBE PENETRATION
SEPARATE AND RESUSPEND CELLS
MIX CELLS WITH FLUORESCENTLY LABELED PROBES AND ALLOW HYBRIDIZATION
FLUORESCENCE MICROSCOPY
PLACE SAMPLE UNDER MICROSCOPE AND OBSERVE INDIVIDUAL CELLS
IMAGE ANALYSIS
PLACE SAMPLE UNDER CAMERA, IRRADIATE AND DETERMINE CELL NUMBER AND / OR FLUORESCENT INTENSITY BY COMPUTER
SEPARATE AND RESUSPEND CELLS
CAPILLARY ELECTROPHORESIS
LOAD SAMPLE INTO ELECTROPHORESIS CHAMBER AND ELECTROPHORETICALLY MOVE THROUGH CAPILLARY AND DETECT BY LASER ENHANCED FLUORESCENCE
FLUOROMETRY
PLACE CELLS INTO A CUVETTE AND MEASURE TOTAL FLUORESCENCE WITH FLUOROMETER
FACS ANALYSIS
RUN SAMPLE THROUGH A FLUORESCENT ACTIVATED CELL SORTER AND COUNT NUMBER OF SIGNALS

FIG. 4

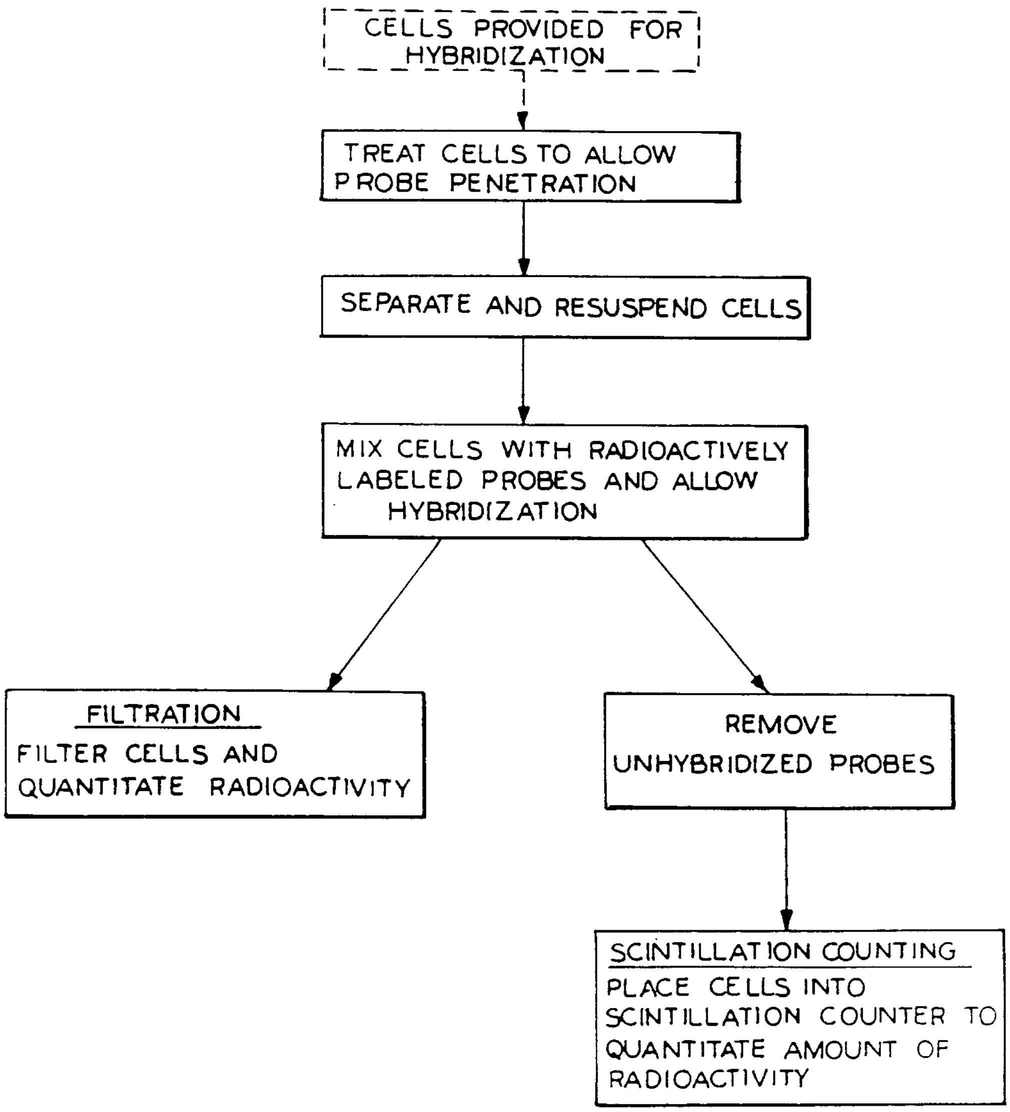
CELLS PROVIDED FOR HYBRIDIZATION
TREAT CELLS TO ALLOW PROBE PENETRATION
SEPARATE AND RESUSPEND CELLS
MIX CELLS WITH RADIOACTIVELY LABELED PROBES AND ALLOW HYBRIDIZATION
FILTRATION
FILTER CELLS AND QUANTITATE RADIOACTIVITY
REMOVE UNHYBRIDIZED PROBES
SCINTILLATION COUNTING
PLACE CELLS INTO SCINTILLATION COUNTER TO QUANTITATE AMOUNT OF RADIOACTIVITY

FIG. 5

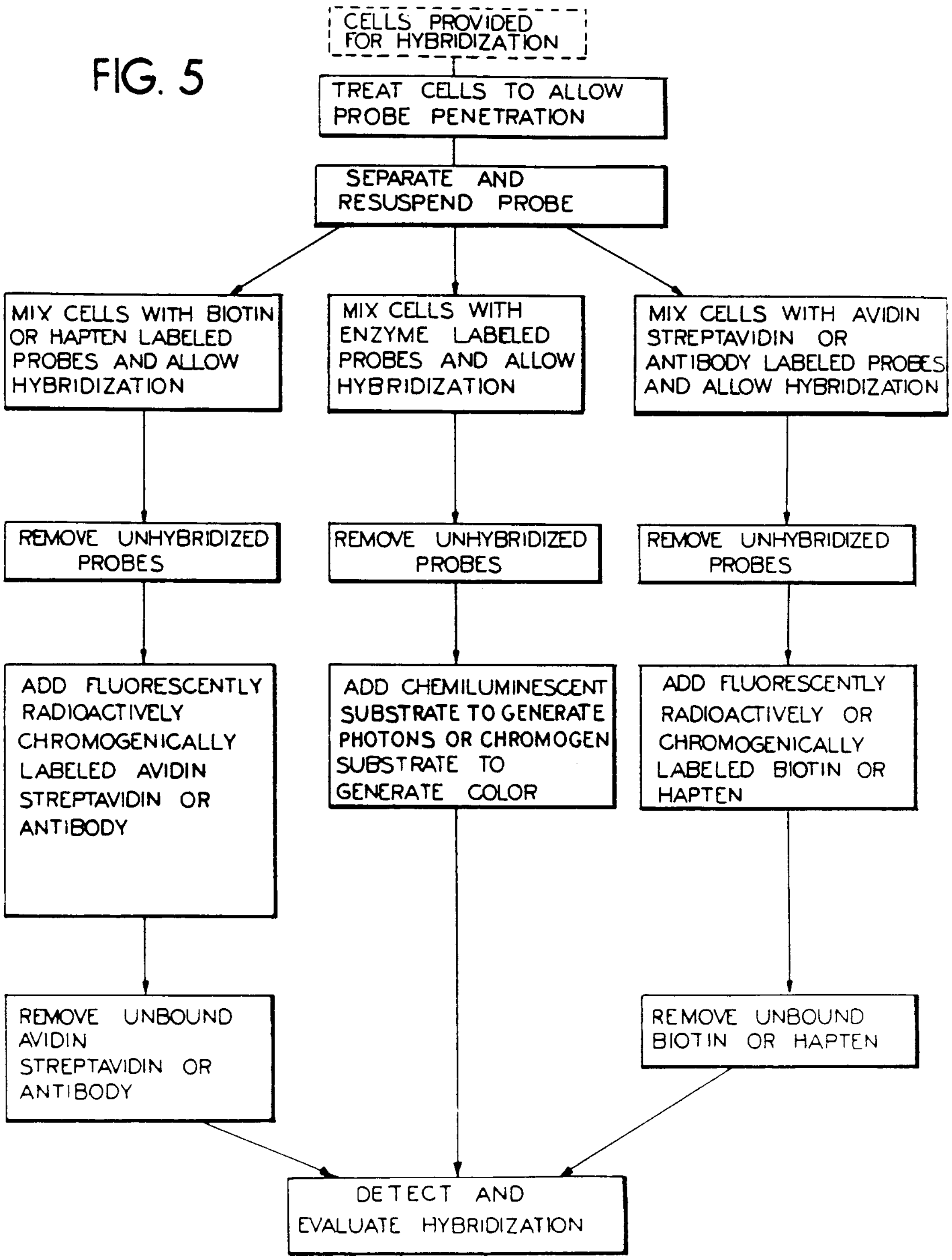
CELLS PROVIDED FOR HYBRIDIZATION
TREAT CELLS TO ALLOW PROBE PENETRATION
SEPARATE AND RESUSPEND PROBE
MIX CELLS WITH BIOTIN OR HAPTEN LABELED PROBES AND ALLOW HYBRIDIZATION
MIX CELLS WITH ENZYME LABELED PROBES AND ALLOW HYBRIDIZATION
MIX CELLS WITH AVIDIN STREPTAVIDIN OR ANTIBODY LABELED PROBES AND ALLOW HYBRIDIZATION
REMOVE UNHYBRIDIZED PROBES
REMOVE UNHYBRIDIZED PROBES
REMOVE UNHYBRIDIZED PROBES
ADD FLUORESCENTLY RADIOACTIVELY CHROMOGENICALLY LABELED AVIDIN STREPTAVIDIN OR ANTIBODY
ADD CHEMILUMINESCENT SUBSTRATE TO GENERATE PHOTONS OR CHROMOGEN SUBSTRATE TO GENERATE COLOR
ADD FLUORESCENTLY RADIOACTIVELY OR CHROMOGENICALLY LABELED BIOTIN OR HAPTEN
REMOVE UNBOUND AVIDIN STREPTAVIDIN OR ANTIBODY
REMOVE UNBOUND BIOTIN OR HAPTEN
DETECT AND EVALUATE HYBRIDIZATION

FIG. 6

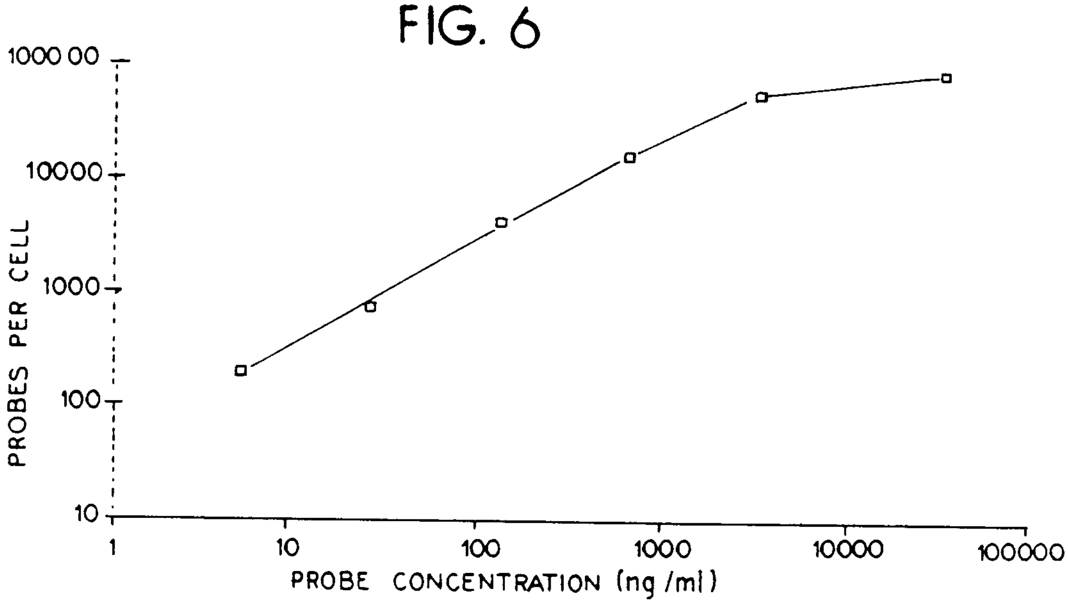


FIG. 7

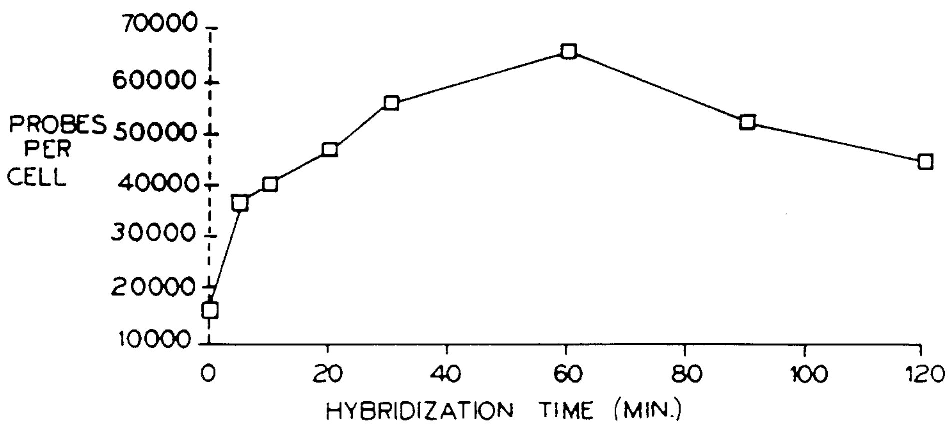


FIG. 8

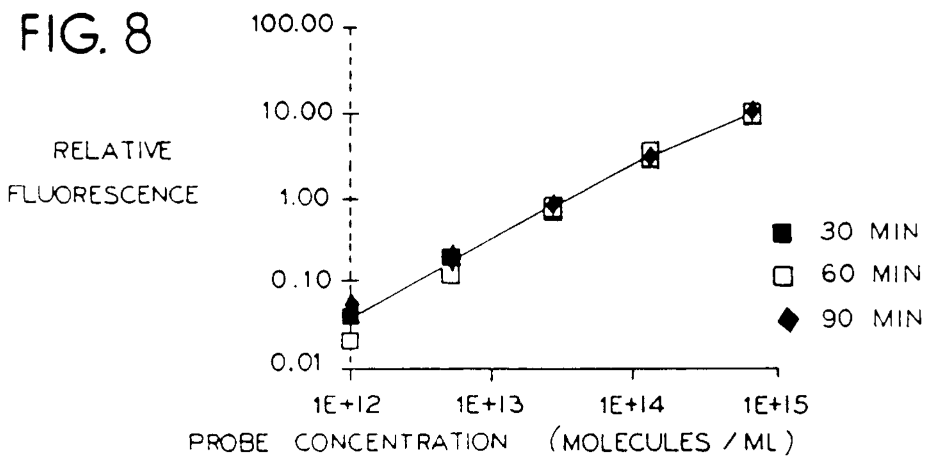

FIG. 9

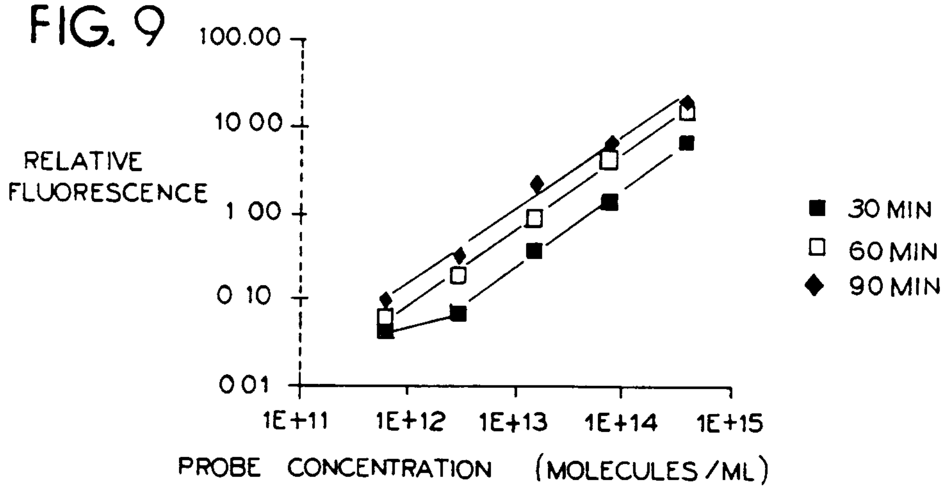
100.00
10.00
1.00
0.10
0.01
RELATIVE FLUORESCENCE
1E+11
1E+12
1E+13
1E+14
1E+15
PROBE CONCENTRATION (MOLECULES/ML)
30 MIN
60 MIN
90 MIN

FIG. 10

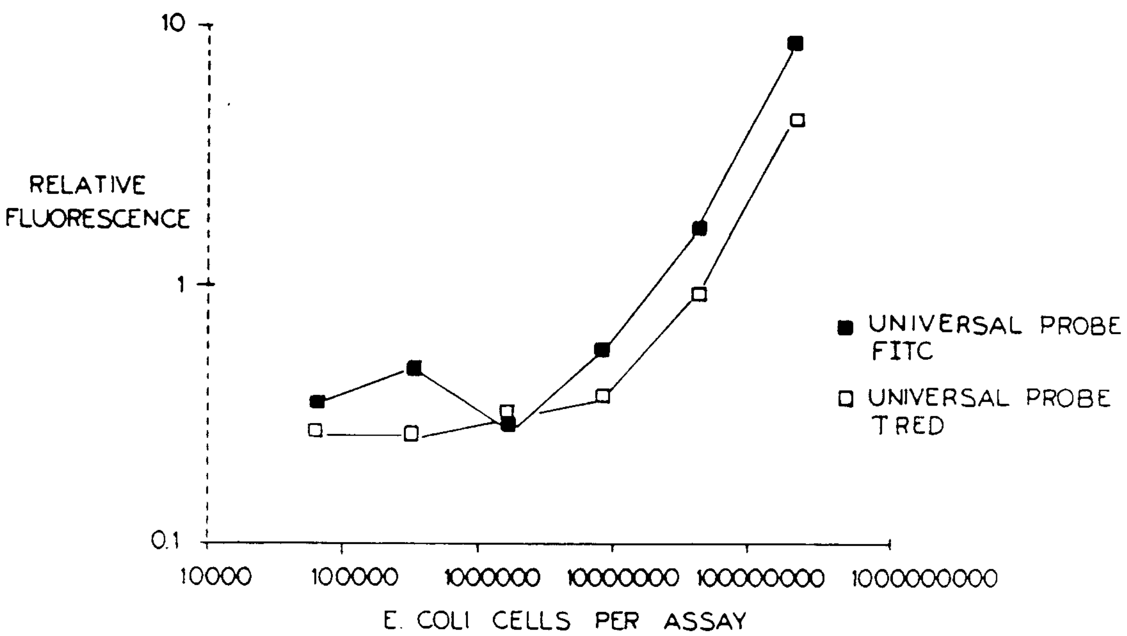
10
1
0.1
RELATIVE FLUORESCENCE
10000
100000
1000000
10000000
100000000
1000000000
E. COLI CELLS PER ASSAY
UNIVERSAL PROBE FITC
UNIVERSAL PROBE TRED

FIG. 11

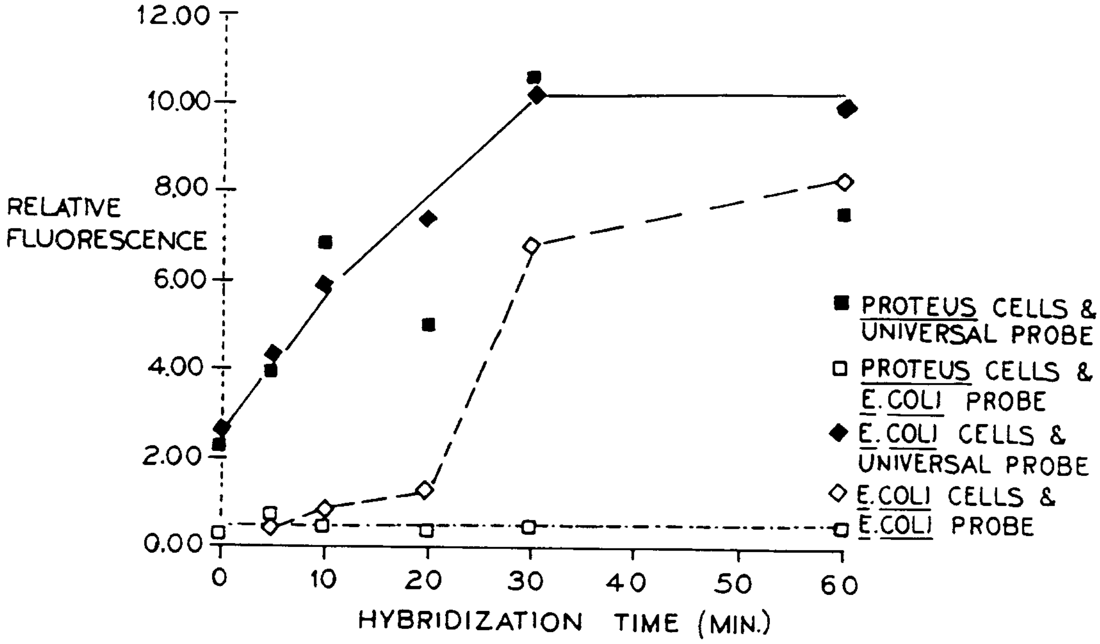
12.00
10.00
8.00
6.00
4.00
2.00
0.00
RELATIVE FLUORESCENCE
0
10
20
30
40
50
60
HYBRIDIZATION TIME (MIN.)
PROTEUS CELLS & UNIVERSAL PROBE
PROTEUS CELLS & E. COLI PROBE
E. COLI CELLS & UNIVERSAL PROBE
E. COLI CELLS & E. COLI PROBE

FIG. 12

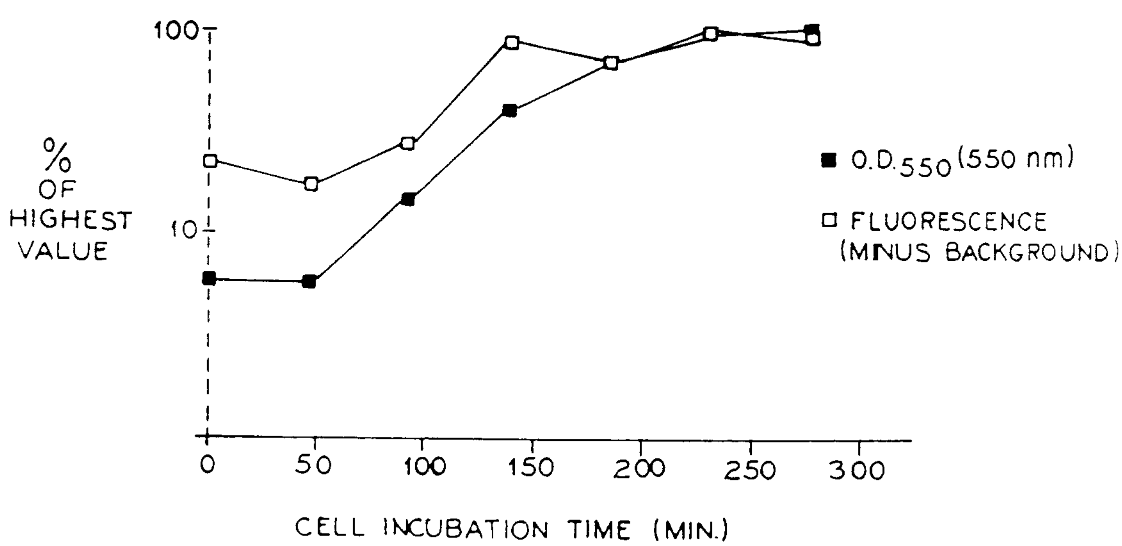
100
10
% OF HIGHEST VALUE
0
50
100
150
200
250
300
CELL INCUBATION TIME (MIN.)
O.D.550 (550 nm)
FLUORESCENCE (MINUS BACKGROUND)

FIG. 13

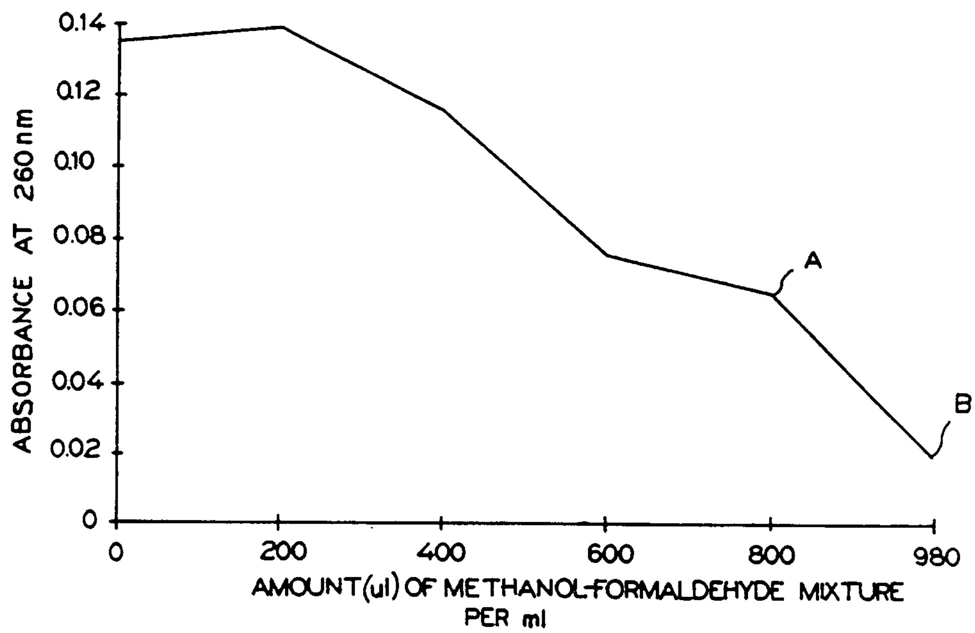

0.14
0.12
0.10
0.08
0.06
0.04
0.02
0
ABSORBANCE AT 260 nm
0
200
400
600
800
980
A
B
AMOUNT (ul) OF METHANOL-FORMALDEHYDE MIXTURE PER ml


FIG. 14

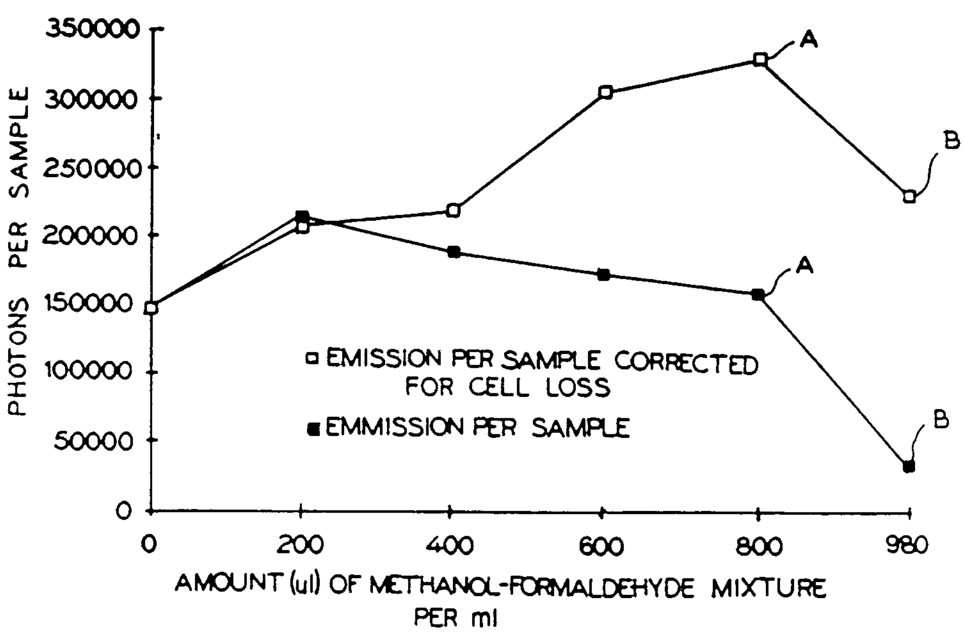

350000
300000
250000
200000
150000
100000
50000
0
PHOTONS PER SAMPLE
0
200
400
600
800
980
A
B
A
B
EMISSION PER SAMPLE CORRECTED FOR CELL LOSS
EMMISSION PER SAMPLE
AMOUNT (ul) OF METHANOL-FORMALDEHYDE MIXTURE PER ml

European Patent Office

# EUROPEAN SEARCH REPORT

Application Number

EP 92300344.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EXPERIMENTAL PARASITOLOGY, vol. 73, no. 3, October 1991, San Diego, G.J. SCHOONE et al. "Detection and Identification of Leishmania Parasites by in situ Hybridization with Total and Recombinant DNA Probes.", pages 345-353 * Abstract * -- | 1,4,6 | C 12 Q 1/68<br>C 12 Q 1/70<br>C 12 Q 1/06 |
| D,A | EP - A - 0 079 139 (ORION CORPORATION LTD) * Abstract; claims * -- | 1 | |
| D,A | SCIENCE, vol. 243, March 10, 1989, Washington, E.F. DELONG et al. "Phylogenetic Stains: Ribosomal RNA-Based Probes for the Identification of Single Cells.", pages 1360-1363 * Abstract * -- | 1,5,6 | TECHNICAL FIELDS SEARCHED (Int. Cl.5)<br>C 12 Q |
| D,A | JOURNAL OF BACTERIOLOGY, vol. 170, no. 2, February 1988, Washington, S.J. GIOVANNONI et al. "Phylogenetic Group-Specific Oligodeoxynucleotide Probes for Identification of Single Microbial Cells.", pages 720-726 * Abstract * ---- | 1,5,6 | |

The present search report has been drawn up for all claims

| Place of search | Date of completion of the search | Examiner |
|---|---|---|
| VIENNA | 12-05-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

SEQUENCE LISTING

(1) GENERAL INFORMATION:

(i) COMPUTER READABLE FORM:

(A) MEDIUM TYPE:
Diskette, 5.25 inch, 360 Kb storage

(B) COMPUTER:
IBM PC/XT Compatible

(C) OPERATING SYSTEM:
MS DOS 3.2

(D) SOFTWARE:
Word Perfect 4.2

(2) INFORMATION FOR SEQ ID NO: 1:

(i) SEQUENCE CHARACTERISTICS:

(A) LENGTH:
18 base pairs

(B) TYPE:
nucleic acid

(C) STRANDEDNESS:
single

(D) TOPOLOGY:
linear

(ii) MOLECULE TYPE:
-rRNA
-cDNA to rRNA

(iii) HYPOTHETICAL:
no

(x) PUBLICATION INFORMATION:

(A) AUTHORS:
Giovannoni, S.J.
DeLong, E.F.
Olsen, G.J.
Pace, N.R.

(B) TITLE:
Phylogenetic Group - Specific Oligodeoxylnucleotide probes for Idenfication of Single Microbial Cells
(C) JOURNAL:
J. Bacteriology
(D) VOLUME:
170
(E) ISSUE:
2
(F) PAGES:
720-726
(G) DATE:
02-1988
(K) RELEVANT RESIDUES IN SEQ ID NO.:
1: 1 to 18

(xi) SEQUENCE DESCRIPTION: SEQ ID NO:1:
GWATTACCGC GGCKGCTG 18

(3) INFORMATION FOR SEQ ID NO: 2:
(i) SEQUENCE CHARACTERISTICS:
(A) LENGTH:
35 base pairs
(B) TYPE:
nucleic acid
(C) STRANDEDNESS:
single
(D) TOPOLOGY:
linear
(ii) MOLECULE TYPE:
-cDNA to rRNA
(iii) HYPOTHETICAL:
no

(xi) SEQUENCE DESCRIPTION: SEQ ID NO:2:

TCAATGAGCA AAGGTATTAA CTTTACTCCC TTCCT 35

(4) INFORMATION FOR SEQ ID NO: 3:

(i) SEQUENCE CHARACTERISTICS:

(A) LENGTH:
28 base pairs

(B) TYPE:
nucleic acid

(C) STRANDEDNESS:
single

(D) TOPOLOGY:
linear

(ii) MOLECULE TYPE:
-cDNA to rRNA

(iii) HYPOTHETICAL:
no

(xi) SEQUENCE DESCRIPTION: SEQ ID NO :3:

AGCTCACAGC ATATGCGCTT TTGTGTAC 28